# EUROPEAN PATENT SPECIFICATION

(11) **EP 4 026 130 B1**
(45) Date of publication and mention of the grant of the patent: **04.11.2026**
(21) Application number: 20776274.1
(22) Date of filing: 08.09.2020
(51) Int. Cl.: G16B 20/00, G16B 30/20, G16B 40/30, C12Q 1/6869

(54) **READ-TIER SPECIFIC NOISE MODELS FOR ANALYZING DNA DATA**
SPEZIFISCHE RAUSCHMODELLE AUF LESEEBENE ZUR ANALYSE VON DNS-DATEN
MODÈLES DE BRUIT SPÉCIFIQUES DE NIVEAU DE LECTURE POUR ANALYSER DES DONNÉES D'ADN

(30) Priority: 09.09.2019 US 201962897923 P
(43) Date of publication of application: 13.07.2022
(73) Proprietor: Grail, Inc., Sunnyvale, CA 94086 (US)
(72) Inventor: HUBBELL, Earl, Menlo Park, CA 94025 (US)
(74) Representative: Murgitroyd & Company
(86) International application number: PCT/US2020/049751
(87) International publication number: WO 2021/050439

(56) References cited:
- WO-A1-2019/169044
- US-A1- 2019 073 445
- KLAMBAUER G ET AL: "cn.MOPS: mixture of Poissons for discovering copy number variations in next-generation sequencing data with a low false discovery rate", NUCLEIC ACIDS RESEARCH, vol. 40, no. 9, 1 February 2012 (2012-02-01), pages e69 - e69, XP055216800, ISSN: 0305-1048, DOI: 10.1093/nar/gks003
- CHRISTOFORIDES A ET AL: "Identification of somatic mutations in cancer through Bayesian-based analysis of sequenced genome pairs", BMC GENOMICS, BIOMED CENTRAL, vol. 14, no. 1, 4 May 2013 (2013-05-04), pages 302, XP021158455, ISSN: 1471-2164, DOI: 10.1186/1471-2164-14-302

## Description

### BACKGROUND

### 1. FIELD OF ART

This disclosure generally relates to noise models for determining quality scores for nucleic acid sequencing datasets.

### 2. DESCRIPTION OF THE RELATED ART

Computational techniques can be used on DNA sequencing data to identify mutations or variants in DNA that may correspond to various types of cancer or other diseases. Thus, cancer diagnosis or prediction may be performed by analyzing a biological sample such as a tissue biopsy or blood drawn from an individual, an aminol, a plant, etc. Detecting DNA that originated from tumor cells from a blood sample is difficult because circulating tumor DNA (ctDNA) is present at low levels relative to other molecules in cell-free DNA (cfDNA) extracted from the blood. The inability of existing methods to identify true positives (e.g., indicative of cancer in the subject) from signal noise diminishes the ability of known and future systems to distinguish true positives from false positives caused by noise sources, which can result in unreliable results for variant calling or other types of analyses.

US 2019/0073445 A1 discloses a method comprising receiving a target read segment and a read frequency of the target read segment, identifying a stratification for the target read segment and accessing a set of parameters specific for the stratification of the received target read segment. The method further comprises applying a significance model comprising two or more distributions to the read frequency of the target read segment to obtain two or more scores, at least one distribution defined by the accessed set of parameters, generating significance information by combining the two or more scores and reporting the target read segment as one of a true or false positive based on the generated significance information.

### SUMMARY

Disclosed herein are systems and methods for training and applying site-specific noise models that are classified into a plurality of read tiers. The noise models can determine likelihoods of true positives in targeted sequencing. True positives can include single nucleotide variants, insertions, or deletions of base pairs. In particular, the models can use Bayesian inference to determine a rate or level of noise, e.g., indicative of an expected likelihood of certain mutations, per position of a nucleic acid sequence. Each model can be specific to a read tier. A read tier can be determined based on whether a potential variant location is located at an overlapping region and/or a complementary region of processed sequencing reads. Each model specific to a read tier can be a hierarchical model that accounts for covariates (e.g., trinucleotide context, mappability, or segmental duplication) and various types of parameters (e.g., mixture components or depth of sequence reads) that are specific to the read tier. The models can be trained from sequence reads of healthy subjects that are also stratified by read tiers. The outputs of different noise models can be combined to generate an overall quality score. An overall pipeline that incorporates various read-tier models can identify true positives at higher sensitivities and filter out false positives when compared to a single model that does not differentiate sequence reads by read tiers.

By way of example, in various embodiments, a method for processing a DNA sequencing dataset of a sample (e.g., an individual) can include accessing the DNA sequencing dataset generated by a DNA sequencing, the DNA sequencing dataset comprising a plurality of processed sequence reads that include a variant location. The method can also include stratifying the plurality of processed sequence reads into a plurality of read tiers. The method can further include determining, for each read tier, a stratified sequencing depth at the variant location. The method can further include determining, for each read tier, one or more noise parameters conditioned on the stratified sequencing depth of the read tier, the one or more noise parameters corresponding to a noise model specific to the read tier. The method can further include generating, for each read tier, an output of the noise model specific to the read tier based on the one or more noise parameters conditioned on the stratified sequencing depth of the read tier. The method can further include combining the generated noise model outputs to produce a combined result. The combined result can represent a likelihood that a total variant count for subsequently observed data being greater than or equal to a total variant count observed in the plurality of processed sequence reads is attributable to noise.

In one or more embodiments, the plurality of read tiers include one or more of: (1) a double-stranded, stitched read tier, (2) a double-stranded, unstitched read tier, (3) a single-stranded, stitched read tier, and (4) a single-stranded, unstitched read tier.

In one or more embodiments, a mutation at the variant location is one of: a single nucleotide variant, an insertion, and a deletion.

In one or more embodiments, the method can further include determining a quality score of the combined result, the quality score being a Phred-scale score.

In one or more embodiments, the method can further include, responsive to the quality score being higher than a predetermined threshold, indicating that the sample is likely to have a mutation at the variant location.

In one or more embodiments, determining, for each read tier, the one or more noise parameters conditioned on the stratified sequencing depth of the read tier can include accessing a parameter distribution specific to the read tier, the parameter distribution describing a distribution of a set of DNA sequencing samples associated with the read tier. The noise parameters are determined from the parameter distribution.

In one or more embodiments, for each read tier, the set of DNA sequencing samples associated with the read tier comprises sequence reads stratified into the read tier and corresponds to one or more healthy individuals.

In one or more embodiments, for each read tier, the noise model specific to the read tier is a Bayesian hierarchical model and the parameter distribution is based on a Gamma distribution.

In one or more embodiments, a first noise parameter corresponding to a noise model specific to a first read tier has a different value than a corresponding second noise parameter corresponding to a noise model specific to a second read tier.

In one or more embodiments, for each read tier, the determined one or more noise parameters comprise a mean of a noise distribution conditioned on the stratified sequencing depth of the read tier.

In one or more embodiments, each noise distribution is a negative binomial distribution conditioned on the stratified sequencing depth of each read tier.

In one or more embodiments for each read tier, the determined one or more noise parameters further comprise a dispersion parameter.

In one or more embodiments, the output for each noise model is the one or more noise parameters conditioned on the stratified sequencing depth determined for the read tier.

In one or more embodiments, the generated output of each noise model is the one or more noise parameters conditioned on the stratified sequencing depth determined for the read tier.

In one or more embodiments, the generated output of each noise model comprises a likelihood that a stratified variant count for the read tier exceeds a threshold.

In one or more embodiments, combining the generated noise model outputs comprises combining a mean variant count and a variance from each noise model output to produce an overall mean variant count and the overall dispersion parameter representative of an overall noise distribution for the combined result.

In one or more embodiments, the overall noise distribution is modeled based on a negative binomial distribution. Determining the overall mean variant count and the overall dispersion parameter can include determining the mean variant count for each read tier based on the stratified sequencing depth of the read tier. The determining step can also include determining the variance for each read tier. The determining step can further include summing the mean variant count for each read tier to determine the overall mean variant count. The determining step can further include combining the variance for each read tier to determine an overall variance. The determining step can further include determining the overall dispersion parameter based on the overall mean variant count and the overall variance.

In one or more embodiments, combining the output for each noise model to generate the combined result can include determining an observed stratified variant count of each read tier. The combining step can also include determining, in each read tier, possible events that are more likely than the observed stratified variant count of each read tier. The combining step can further include identifying combinations of the possible events associated with a higher likelihood of occurrence than the observed stratified variant count of each read tier. The combining step can further include summing probabilities of the identified combinations to determine a statistic complement. The combining step can further include determining a likelihood value by subtracting the statistic complement from 1.0.

In one or more embodiments, a first identified combination comprising one double-stranded read is equivalent to a second identification combination comprising two single-stranded reads.

In one or more embodiments, the determined likelihood value is equal to or greater than a likelihood of occurrence of the observed stratified variant count of each read tier.

In one or more embodiments, the method can further include training a machine learning model to determine the likelihood value.

In one or more embodiments, the method can further include receiving a body fluid sample of the individual. The method can further include performing the DNA sequencing on cfDNA of the body fluid sample. The method can further include generating raw sequence reads based on a result of the DNA sequencing. The method can further include collapsing and stitching the raw sequence reads to generate the plurality of processed sequence reads.

In one or more embodiments, the body fluid sample is a sample of one of: blood, whole blood, plasma, serum, urine, cerebrospinal fluid, fecal, saliva, tears, a tissue biopsy, pleural fluid, pericardial fluid, or peritoneal fluid of the individual.

In one or more embodiments, the plurality of processed sequence reads is sequenced from a tumor biopsy.

In one or more embodiments, the plurality of processed sequence reads is sequenced from an isolate of cells from blood, the isolate of cells including at least buffy coat white blood cells or CD4+ cells.

In one or more embodiments, the DNA sequencing is a type of massively parallel DNA sequencing.

In various embodiments, a non-transitory computer readable medium includes instructions that, when executed by one or more processors, cause the one or more processors to perform any of the steps described above and disclosed herein.

Further, in various embodiments, a system having a computer processor and a memory that stores computer program instructions is provided, whereby executing the instructions by the computer processor cause the processor to perform any of the steps described above and disclosed herein.

Embodiments according to the invention are in particular disclosed in the attached claims directed to a method and a computer program product, wherein any feature mentioned in one claim category, e.g. method, can be claimed in another claim category, e.g. computer program product, system, storage medium, as well. The dependencies or references back in the attached claims are chosen for formal reasons only. However, any subject matter resulting from a deliberate reference back to any previous claims (in particular multiple dependencies) can be claimed as well, so that any combination of claims and the features thereof is disclosed and can be claimed regardless of the dependencies chosen in the attached claims. The subject-matter which can be claimed comprises not only the combinations of features as set out in the attached claims but also any other combination of features in the claims, wherein each feature mentioned in the claims can be combined with any other feature or combination of other features in the claims. Furthermore, any of the embodiments and features described or depicted herein can be claimed in a separate claim and/or in any combination with any embodiment or feature described or depicted herein or with any of the features of the attached claims.

### BRIEF DESCRIPTION OF DRAWINGS

FIG. 1 is a flowchart of a method for preparing a nucleic acid sample for sequencing according to various embodiments of the present disclosure.
FIG. 2 is a block diagram of a processing system for processing sequence reads according to various embodiments of the present disclosure.
FIG. 3 is a flowchart of a method for determining variants of sequence reads according to various embodiments of the present disclosure.
FIG. 4 is a diagram of an application of a Bayesian hierarchical model according to various embodiments of the present disclosure.
FIG. 5A shows dependencies between parameters and sub-models of a Bayesian hierarchical model for determining true single nucleotide variants according to various embodiments of the present disclosure.
FIG. 5B shows dependencies between parameters and sub-models of a Bayesian hierarchical model for determining true insertions or deletions according to various embodiments of the present disclosure.
FIG. 6A illustrates a diagram for a distribution of noise rates associated with a Bayesian hierarchical model according to various embodiments of the present disclosure.
FIG. 6B illustrates a diagram for a distribution of variant given parameters associated with a Bayesian hierarchical model according to various embodiments of the present disclosure.
FIG. 7A is a diagram of determining parameters by fitting a Bayesian hierarchical model according to various embodiments of the present disclosure.
FIG. 7B is a diagram of using parameters from a Bayesian hierarchical model to determine a likelihood of a false positive according to various embodiments of the present disclosure.
FIG. 8A includes illustrating different read tiers of sequence reads, according to various embodiments of the present disclosure.
FIG. 8B shows an experimental result that illustrates different qualities of the read tiers of FIG. 8A, according to various embodiments of the present disclosure.
FIG. 8C shows experimental results of a first read tier obtained by stratifying the sequence reads into read tiers and further into sub-read tiers based on the types of nucleotide substitution, according to various embodiments of the present disclosure.
FIG. 8D shows experimental results of a second read tier obtained by stratifying the sequence reads into read tiers and further into sub-read tiers based on the types of nucleotide substitution, according to various embodiments of the present disclosure.
FIG. 8E shows experimental results of a third read tier obtained by stratifying the sequence reads into read tiers and further into sub-read tiers based on the types of nucleotide substitution, according to various embodiments of the present disclosure.
FIG. 8F shows experimental results of a fourth read tier obtained by stratifying the sequence reads into read tiers and further into sub-read tiers based on the types of nucleotide substitution, according to various embodiments of the present disclosure.
FIG. 8G shows experimental results of a fifth read tier obtained by stratifying the sequence reads into read tiers and further into sub-read tiers based on the types of nucleotide substitution, according to various embodiments of the present disclosure.
FIG. 8H shows mean error rates across the four read tiers based on types of alternate alleles, according to various embodiments of the present disclosure.
FIG. 9 is a flowchart depicting a process for analyzing a DNA sequencing sample using stratified noise models according to various embodiments of the present disclosure.
FIG. 10 is a flowchart depicting a process for combining outputs of stratified noise models for different read tiers using moment matching according to various embodiments of the present disclosure.
FIG. 11A is a flowchart depicting a process for combining outputs of stratified noise models for different read tiers using integration according to various embodiments of the present disclosure.
FIG. 11B illustrates the counting of more extreme events in a multi-dimensional space according to various embodiments of the present disclosure.
FIG. 12A illustrates an example plot of observed quality scores against default quality scores according to various embodiments of the present disclosure.
FIG. 12B illustrates another example plot of observed quality scores against default quality scores according to various embodiments of the present disclosure.
FIG. 13A illustrates experimental results of quality scores using read tiers according to various embodiments of the present disclosure.
FIG. 13B illustrates experimental results of quality scores using a noise model that does not differentiate read tiers according to various embodiments of the present disclosure.
FIG. 14 is a flowchart depicting an example process that identifies potential mutation locations of an individual according to various embodiments of the present disclosure.
FIG. 15 is a block diagram of an example computing device according to various embodiments of the present disclosure.

The figures depict embodiments of the present invention for purposes of illustration only.

### DETAILED DESCRIPTION

### I. DEFINITIONS

The term "individual" refers to a human individual. The term "healthy individual" refers to an individual presumed to not have cancer or a disease. The term "subject" refers to an individual who is being tested for cancer or a disease.

The term "sequence reads" refers to nucleotide sequences read from a sample obtained from an individual. Sequence reads can be obtained through various methods known in the art.

The term "read segment" or "read" refers to any nucleotide sequences including sequence reads obtained from an individual and/or nucleotide sequences derived from the initial sequence read from a sample obtained from an individual. For example, a read segment can refer to an aligned sequence read, a collapsed sequence read, or a stitched read. Furthermore, a read segment can refer to an individual nucleotide base, such as a single nucleotide variant.

The term "single nucleotide variant" or "SNV" refers to a substitution of one nucleotide to a different nucleotide at a position (e.g., site) of a nucleotide sequence, e.g., a sequence read from an individual. A substitution from a first nucleobase X to a second nucleobase Y can be denoted as "X>Y." For example, a cytosine to thymine SNV may be denoted as "C>T."

The term "indel" refers to any insertion or deletion of one or more base pairs having a length and a position (which can also be referred to as an anchor position) in a sequence read. An insertion corresponds to a positive length, while a deletion corresponds to a negative length.

The term "variant" refers to one or more SNVs or indels. A variant location refers to a location of interest in a DNA sequencing that could potentially contain SNVs or indels.

The term "true positive" refers to a mutation that indicates real biology, for example, the presence of a potential cancer, disease, or germline mutation in an individual. True positives are not caused by mutations naturally occurring in healthy individuals (e.g., recurrent mutations) or other sources of artifacts such as process errors during assay preparation of nucleic acid samples.

The term "false positive" refers to a mutation incorrectly determined to be a true positive. Generally, false positives can be more likely to occur when processing sequence reads associated with greater mean noise rates or greater uncertainty in noise rates.

The term "cell-free DNA" or "cfDNA" refers to nucleic acid fragments that circulate in an individual's body (e.g., bloodstream) and originate from one or more healthy cells and/or from one or more cancer cells.

The term "circulating tumor DNA" or "ctDNA" refers to nucleic acid fragments that originate from tumor cells or other types of cancer cells, which can be released into an individual's bloodstream as a result of biological processes such as apoptosis or necrosis of dying cells or actively released by viable tumor cells.

The term "alternative allele" or "ALT" refers to an allele having one or more mutations relative to a reference allele, e.g., corresponding to a known gene.

The term "sequencing depth" or "depth" refers to a total number of read segments from a sample obtained from an individual.

The term "alternate depth" or "AD" refers to a number of read segments in a sample that supports an ALT, e.g., include mutations of the ALT.

The term "alternate frequency" or "AF" refers to the frequency of a given ALT. The AF can be determined by dividing the corresponding AD of a sample by the depth of the sample for the given ALT.

### II. EXAMPLE ASSAY PROTOCOL

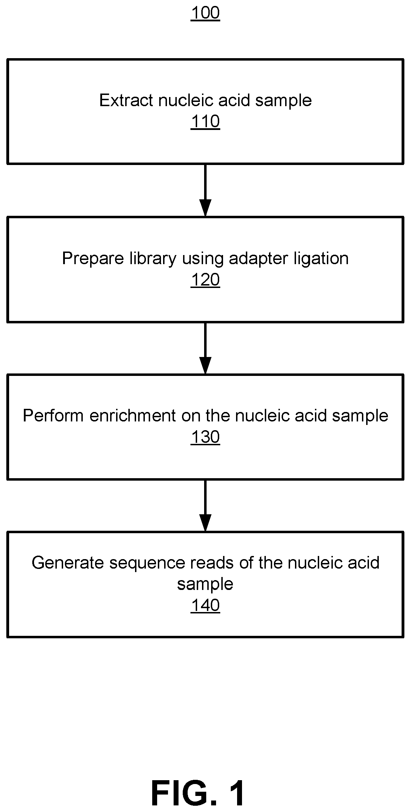

FIG. 1 is a flowchart of a method 100 for preparing a nucleic acid sample for sequencing according to various embodiments. The method 100 includes, but is not limited to, the following steps. For example, any step of the method 100 can comprise a quantitation sub-step for quality control or other laboratory assay procedures known to one skilled in the art. The method 100 can correspond to a type of massively parallel DNA sequencing, for example, a next-generation sequencing (NGS).

In step 110, a nucleic acid sample (DNA or RNA) is extracted from a subject. The subject can be an individual. The sample can be any subset of the human genome or the whole genome. The sample can be extracted from a subject known to have or suspected of having cancer. The sample can include blood, plasma, serum, urine, fecal, saliva, other types of bodily fluids, or any combination thereof. In some embodiments, methods for drawing a blood sample (e.g., syringe or finger prick) can be less invasive than procedures for obtaining a tissue biopsy, which can require surgery. The extracted sample can comprise cfDNA and/or ctDNA. For healthy individuals, the human body can naturally clear out cfDNA and other cellular debris. If a subject has cancer or a disease, ctDNA in an extracted sample can be present at a detectable level for diagnosis.

In step 120, a sequencing library is prepared. During the library preparation, the nucleic acid samples are randomly cleaved into thousands or millions of fragments. Unique molecular identifiers (UMI) are added to the nucleic acid fragments (e.g., DNA fragments) through adapter ligation. The UMIs are short nucleic acid sequences (e.g., 4-10 base pairs) that are added to ends of DNA fragments during adapter ligation. In some embodiments, UMIs are degenerate base pairs that serve as a unique tag that can be used to identify sequence reads originating from a specific DNA fragment. During PCR amplification following adapter ligation, the UMIs are replicated along with the attached DNA fragment, which provides a way to identify sequence reads that came from the same original fragment in downstream analysis.

In step 130, targeted DNA sequences are enriched from the library. During enrichment, hybridization probes (also referred to herein as "probes") are used to target, and pull down, nucleic acid fragments informative for the presence or absence of cancer (or disease), cancer status, or a cancer classification (e.g., cancer type or tissue of origin). For a given workflow, the probes can be designed to anneal (or hybridize) to a target (complementary) strand of DNA or RNA. The target strand can be the "positive" strand (e.g., the strand transcribed into mRNA, and subsequently translated into a protein) or the complementary "negative" strand. The probes can range in length from 10s, 100s, or 1000s of base pairs. In some embodiments, the probes are designed based on a gene panel to analyze particular mutations or target regions of the genome (e.g., of the human or another organism) that are suspected to correspond to certain cancers or other types of diseases. Moreover, the probes can cover overlapping portions of a target region. By using a targeted gene panel rather than sequencing all expressed genes of a genome, also known as "whole exome sequencing," the method 100 can be used to increase the sequencing depth of the target regions, where depth refers to the count of the number of times a given target sequence within the sample has been sequenced. Increasing sequencing depth reduces the required input amounts of the nucleic acid sample. After a hybridization step, the hybridized nucleic acid fragments are captured and can also be amplified using PCR.

In step 140, sequence reads are generated from the enriched DNA sequences. Sequencing data can be acquired from the enriched DNA sequences by known means in the art. For example, the method 100 can include next-generation sequencing (NGS) techniques including synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators.

In some embodiments, the sequence reads can be aligned to a reference genome using known methods in the art to determine the alignment position information. The alignment position information can indicate a beginning position and an end position of a region in the reference genome that corresponds to a beginning nucleotide base and end nucleotide base of a given sequence read. Alignment position information can also include sequence read length, which can be determined from the beginning position and end position. A region in the reference genome can be associated with a gene or a segment of a gene.

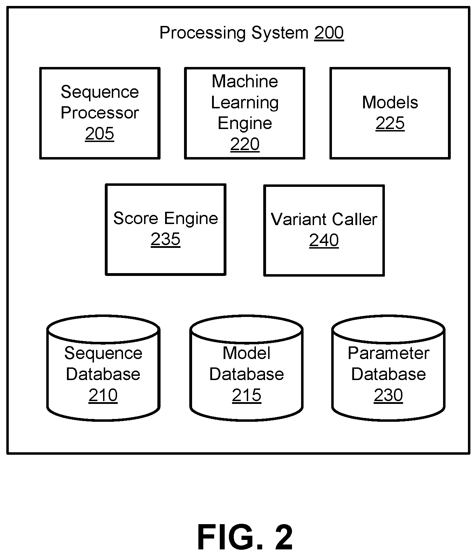

In various embodiments, a sequence read is comprised of a read pair denoted as *R*₁ and *R*₂. For example, the first read *R*₁ can be sequenced from a first end of a nucleic acid fragment whereas the second read *R*₂ can be sequenced from the second end of the nucleic acid fragment. Therefore, nucleotide base pairs of the first read *R*₁ and second read *R*₂ can be aligned consistently (e.g., in opposite orientations) with nucleotide bases of the reference genome. Alignment position information derived from the read pair *R*₁ and *R*₂ can include a beginning position in the reference genome that corresponds to an end of a first read (e.g., *R*₁) and an end position in the reference genome that corresponds to an end of a second read (e.g., *R*₂). In other words, the beginning position and end position in the reference genome represent the likely location within the reference genome to which the nucleic acid fragment corresponds. An output file having SAM (sequence alignment map) format or BAM (binary) format can be generated and output for further analysis such as variant calling, as described below with respect to FIG. 2.

### III. EXAMPLE PROCESSING SYSTEM

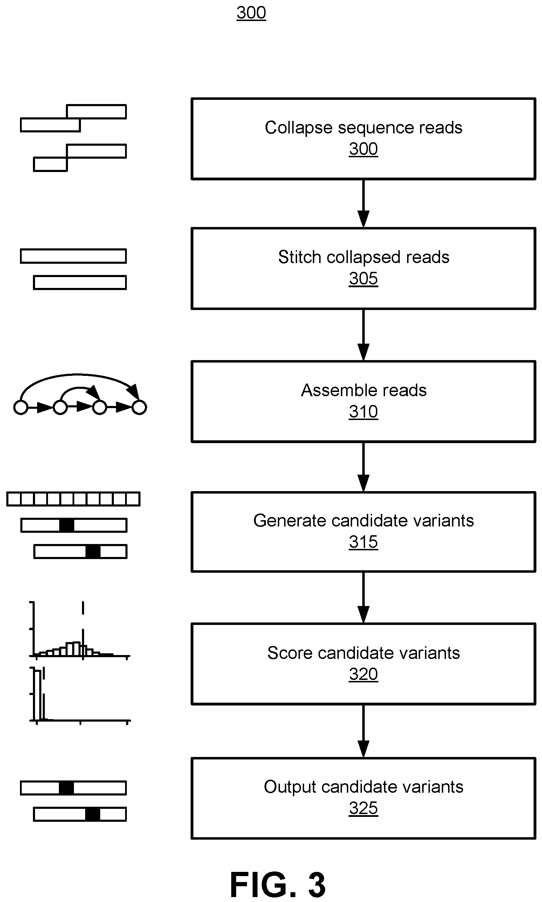

FIG. 2 is a block diagram of a processing system 200 for processing sequence reads according to various embodiments. The processing system 200 includes a sequence processor 205, sequence database 210, model database 215, machine learning engine 220, models 225 (for example, Bayesian hierarchical models that correspond to different read tiers), parameter database 230, score engine 235, and variant caller 240. FIG. 3 is a flowchart of a method 300 for determining variants of sequence reads according to various embodiments. In some embodiments, the processing system 200 performs the method 300 to perform variant calling (e.g., for SNVs and/or indels) based on input sequencing data. Further, the processing system 200 can obtain the input sequencing data from an output file associated with a nucleic acid sample prepared using the method 100 described above. The method 300 includes, but is not limited to, the following steps, which are described with respect to the components of the processing system 200. In some embodiments, one or more steps of the method 300 can be replaced by a step of a different process for generating variant calls, e.g., using Variant Call Format (VCF), such as HaplotypeCaller, VarScan, Strelka, or SomaticSniper.

In step 300, the sequence processor 205 collapses sequence reads of the input sequencing data. In some embodiments, collapsing sequence reads includes using UMIs, and optionally alignment position information from sequencing data of an output file (e.g., from the method 100 shown in FIG. 1) to collapse multiple sequence reads into a consensus sequence for determining the most likely sequence of a nucleic acid fragment or a portion thereof. Because the UMIs are replicated with the ligated nucleic acid fragments through enrichment and PCR, the sequence processor 205 can determine that certain sequence reads originated from the same molecule in a nucleic acid sample. In some embodiments, sequence reads that have the same or similar alignment position information (e.g., beginning and end positions within a threshold offset) and include a common UMI are collapsed, and the sequence processor 205 generates a collapsed read (also referred to herein as a consensus read) to represent the nucleic acid fragment. The sequence processor 205 designates a consensus read as "duplex" if the corresponding pair of collapsed reads have a common UMI, which indicates that both positive and negative strands of the originating nucleic acid molecule are captured; otherwise, the collapsed read is designated "non-duplex." In some embodiments, the sequence processor 205 can perform other types of error correction on sequence reads as an alternative to, or in addition to, collapsing sequence reads.

In step 305, the sequence processor 205 stitches the collapsed reads based on portions of overlapping nucleotide sequences among two or more sequence reads. In some embodiments, the sequence processor 205 compares nucleotide sequences between a first read and a second read to determine whether nucleotide base pairs of the first and second reads overlap. The two sequence reads can also be compared to a reference genome. In an example use case, responsive to determining that an overlap (e.g., of a given number of nucleotide bases) between the first and second reads is greater than a threshold length (e.g., a threshold number of nucleotide bases), the sequence processor 205 designates the first and second reads as "stitched"; otherwise, the collapsed reads are designated "unstitched." In some embodiments, the first and second reads are stitched if the overlap is greater than the threshold length and if the overlap is not a sliding overlap. For example, a sliding overlap can include a homopolymer run (e.g., a single repeating nucleotide base), a dinucleotide run (e.g., two-nucleotide base sequence), or a trinucleotide run (e.g., three-nucleotide base sequence), where the homopolymer run, dinucleotide run, or trinucleotide run has at least a threshold length of base pairs.

In step 310, the sequence processor 205 assembles reads into paths. In some embodiments, the sequence processor 205 assembles reads to generate a directed graph, for example, a de Bruijn graph, for a target region (e.g., a gene). Unidirectional edges of the directed graph represent sequences of k nucleotide bases (also referred to herein as "k-mers") in the target region, and the edges are connected by vertices (or nodes). The sequence processor 205 aligns collapsed reads to a directed graph such that any of the collapsed reads can be represented in order by a subset of the edges and corresponding vertices.

In some embodiments, the sequence processor 205 determines sets of parameters describing directed graphs and processes directed graphs. Additionally, the set of parameters can include a count of successfully aligned k-mers from collapsed reads to a k-mer represented by a node or edge in the directed graph. The sequence processor 205 stores, e.g., in the sequence database 210, directed graphs and corresponding sets of parameters, which can be retrieved to update graphs or generate new graphs. For instance, the sequence processor 205 can generate a compressed version of a directed graph (e.g., or modify an existing graph) based on the set of parameters. In an example use case, in order to filter out data of a directed graph having lower levels of importance, the sequence processor 205 removes (e.g., "trims" or "prunes") nodes or edges having a count less than a threshold value, and maintains nodes or edges having counts greater than or equal to the threshold value.

In step 315, the variant caller 240 generates candidate variant reads from the paths assembled by the sequence processor 205. A variant can correspond to an SNV or an indel. In some embodiments, the variant caller 240 can generate the candidate variant reads by comparing a directed graph (which could have been compressed by pruning edges or nodes in step 310) to a reference sequence of a target region of a genome. The variant caller 240 can align edges of the directed graph to the reference sequence, and records the genomic positions of mismatched edges and mismatched nucleotide bases adjacent to the edges as the locations of candidate variants. Additionally, the variant caller 240 can generate candidate variant reads based on the sequencing depth of a target region. In particular, the variant caller 240 can be more confident in identifying variants in target regions that have greater sequencing depth, for example, because a greater number of sequence reads help to resolve (e.g., using redundancies) mismatches or other base pair variations between sequences.

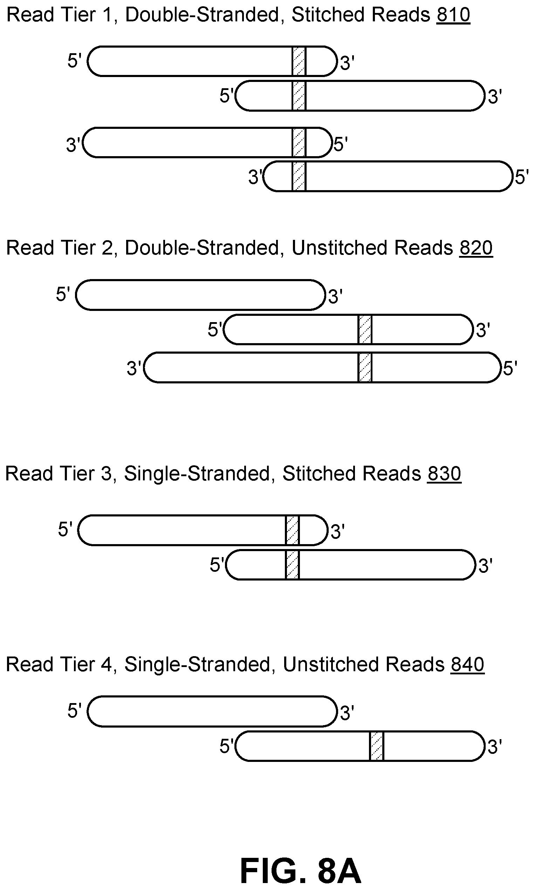

In some embodiments, variant reads can be classified into different read tiers, based on the quality of the variant read. The quality of the variant read can correspond to the location of the potential variant location compared to the overlap and/or complementary locations of the collapsed sequences. In a sample preparation (e.g., a library preparation process) in a massively parallel sequencing, the nucleic acid samples of a subject individual can be cleaved randomly and before parallel sequencing is performed. The same copies of a nucleic acid sequence can be cleaved differently and randomly. Hence, some of the enriched fragments can have overlapped regions that can be stitched with other enriched fragments, while other enriched fragments do not. Some enriched fragments can also have complementary sequences that are also enriched, thus generating a double-stranded fragment in sequence processing. As a result, variant reads for different sequence locations can correspond to different qualities. For example, a variant read at a location in which both complementary strands of fragments are enriched often has better quality than another variant read at another location that only finds support from a single-stranded fragment. The details of the read tiers of the variant reads will be further discussed in FIGS. 8A-8B.

In some embodiments, the variant caller 240 generates candidate variant reads using the models 225 to determine expected noise rates for sequence reads from a subject. Each of the models 225 can be a Bayesian hierarchical model. A Bayesian hierarchical model can be one of many possible model architectures that can be used to generate candidate variants and which are related to each other in that they all model position-specific noise information in order to improve the sensitivity/specificity of variant calling. More specifically, the machine learning engine 220 trains the model 225 using samples from healthy individuals to model the expected noise rates per position of sequence reads. In some embodiments, the variant reads corresponding to different read tiers can be treated differently by different models each specific to a particular read tier. The results of each model can be combined to generate a combined result. The details of stratifying read tiers and models will be further discussed below in association with FIGS. 8A-11B.

Further, multiple different models can be stored in the model database 215 or retrieved for application post-training. For example, a first model is trained to model SNV noise rates and a second model is trained to model indel noise rates. Further, the score engine 235 can use parameters of the models 225 to determine a likelihood of one or more true positives in a sequence read. The score engine 235 can determine a quality score (e.g., on a logarithmic scale) based on the likelihood. For example, the quality score is a Phred quality score *Q* = -10 · *log*₁₀ *P* , where P is the likelihood of an incorrect candidate variant call (e.g., a false positive).

In step 320, the score engine 235 scores the variant reads based on the models 225 or corresponding likelihoods of true positives or quality scores. Training and application of the model 225 are described in more detail below.

In step 325, the processing system 200 outputs the analysis result with respect to the variants. In some embodiments, the processing system 200 outputs some or all of the determined candidate variants along with the corresponding scores. Downstream systems, e.g., external to the processing system 200 or other components of the processing system 200, can use the variants and scores for various applications including, but not limited to, predicting the presence of cancer, disease, or germline mutations.

### IV. EXAMPLE MODELS

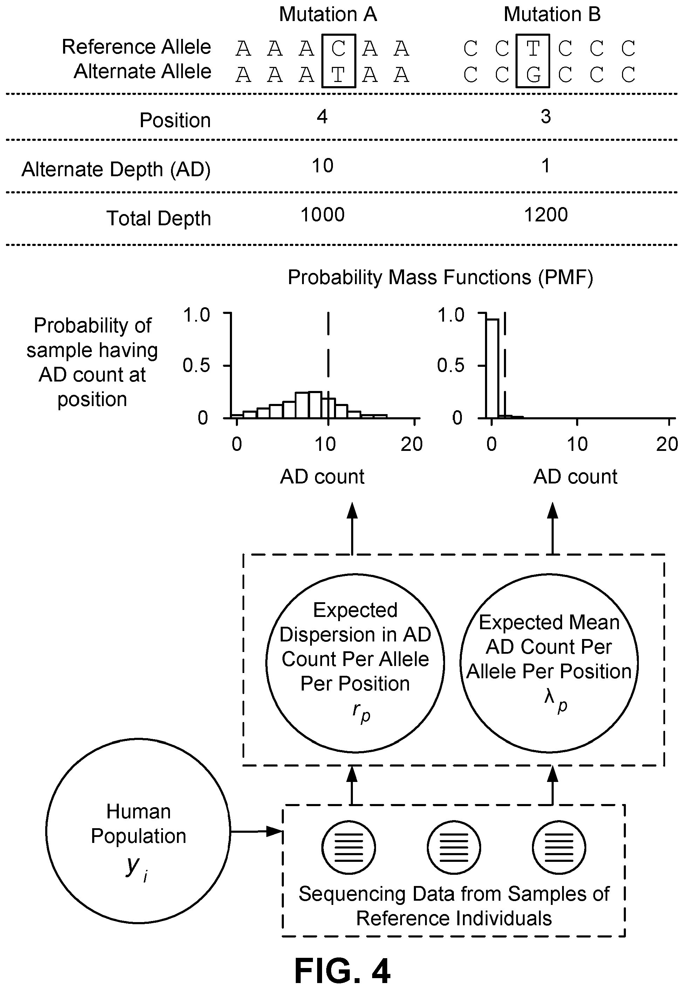

FIG. 4 is a diagram of an application of a Bayesian hierarchical model 225 according to various embodiments. Mutation A and Mutation B are shown as examples for purposes of explanation. In the majority of this disclosure including in FIG. 4, mutations are represented as SNVs, though in some embodiments, the description in this disclosure is also applicable to indels or other types of mutations. A first variant read of a first sample corresponds to an example mutation A, which is a C>T mutation at position 4 of a first reference allele. The first sample has a first AD of 10 and a first total sequencing depth of 1000. A second variant read of a second sample corresponds to an example mutation B, which is a T>G mutation at position 3 of a second reference allele. The second sample has a second AD of 1 and a second total depth of 1200. Based merely on AD (or AF), Mutation A may appear to be a true positive, while Mutation B may appear to be a false positive because the AD (or AF) of the former is greater than that of the latter. However, Mutations A and B can have different relative levels of noise rates per allele and/or per position of the allele. For example, Mutation A can be a false positive and Mutation B can be a true positive, once the relative noise levels of these different positions are accounted for. The models 225 described herein model this noise for appropriate identification of true positives accordingly.

The probability mass functions (PMFs) illustrated in FIG. 4 indicate the probability (or likelihood) of a sample from a subject having a given AD count at a position. Using sequencing data from samples of reference individuals (e.g., stored in the sequence database 210) as training datasets, the processing system 200 trains models 225 from which the PMFs for reference samples can be derived. The reference individuals can be individuals who are not known or found to be associated with a mutation at the specific variant location and can sometimes be referred to as healthy individuals, although the healthy individual can be associated with a different mutation in another variant location that is unrelated to the model trained for the specific variant location. The PMFs are based on *λₚ*, which models the expected mean AD count per allele per position in normal tissue (e.g., of a reference individual), and *rₚ*, which models the expected variation (e.g., dispersion) in this AD count. Stated differently, *λₚ* and/or *rₚ* represent a baseline level of noise, on a per position per allele basis, in the sequencing data for normal tissue. In some embodiments, the sequencing data of references individuals can be stratified into different read tiers so that multiple models 225 each corresponding to a particular read tier are trained. Each model corresponding to a read tier can have a different *λₚ* and a different *rₚ*.

Using the example of FIG. 4 to further illustrate, samples from the reference individuals represent a subset of the human population modeled by *yᵢ*, where *i* is the index of the healthy individual in the training set. Assuming for sake of example a model 225 has already been trained, PMFs produced by the model 225 visually illustrate the likelihood of the measured ADs for each mutation, and therefore provide an indication of which are true positives and which are false positives. The example PMF on the left of FIG. 4 associated with Mutation A indicates that the probability of the first sample having an AD count of 10 for the mutation at position 4 is approximately 20%. Additionally, the example PDF on the right associated with Mutation B indicates that the probability of the second sample having an AD count of 1 for the mutation at position 3 is approximately 1% (note: the PMFs of FIG. 4 are not exactly to scale). Thus, the noise rates corresponding to these probabilities of the PMFs indicate that Mutation A is more likely to occur than Mutation B, despite Mutation B having a lower AD and AF. Thus, in this example, Mutation B can be the true positive and Mutation A can be the false positive. Accordingly, the processing system 200 can perform improved variant calling by using the model 225 to distinguish true positives from false positives at a more accurate rate, and further provide numerical confidence as to these likelihoods.

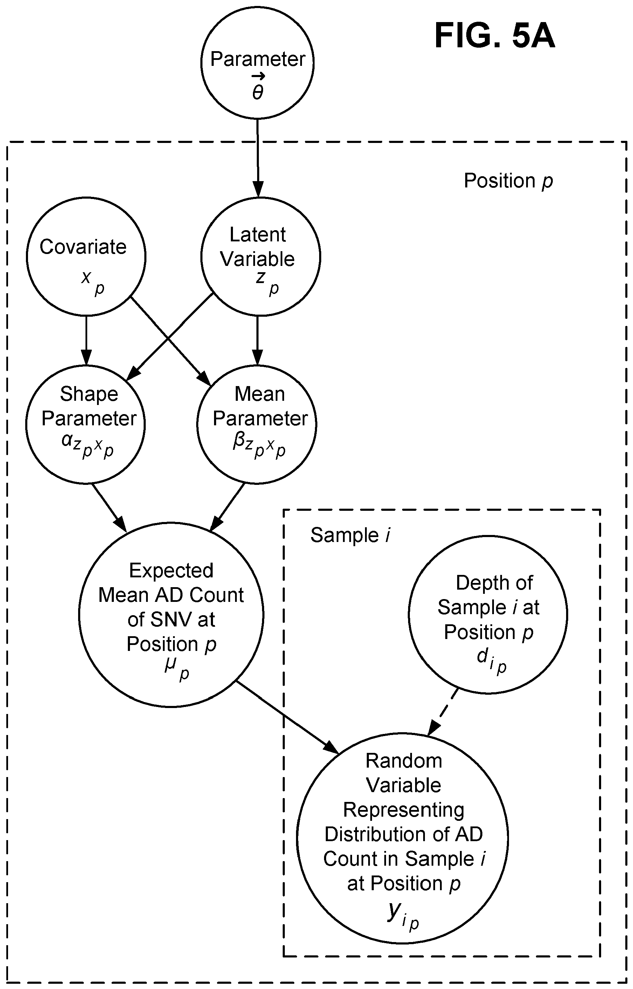

FIG. 5A shows dependencies between parameters and sub-models of a Bayesian hierarchical model 225 for determining true single nucleotide variants according to various embodiments. The Bayesian hierarchical model shown in FIG. 5A can correspond to a specific read tier of a variant read. Parameters of models can be stored in the parameter database 230. In the example shown in FIG. 5A, *̅θ̅*̅ represents the vector of weights assigned to each mixture component. The vector *̅θ̅*̅ takes on values within the simplex in K dimensions and can be learned or updated via posterior sampling during training. It can be given a uniform prior on said simplex for such training. The mixture component to which a position *p* belongs can be modeled by a latent variable *zₚ* using one or more different multinomial distributions: ${z}_{p} ∼ Multinom \left(\vec{θ}\right)$

Together, the latent variable *zₚ*, the vector of mixture components *̅θ̅*̅, *α*, and *β* allow the model for *µ*, that is, a sub-model of the Bayesian hierarchical model 225, to have parameters that "pool" knowledge about noise, that is they represent similarity in noise characteristics across multiple positions. Thus, positions of sequence reads can be pooled or grouped into latent classes by the model. Also advantageously, samples of any of these "pooled" positions can help train these shared parameters. A benefit of this is that the processing system 200 can determine a model of noise in healthy samples even if there is little to no direct evidence of alternate alleles having been observed for a given position previously (e.g., in the healthy tissue samples used to train the model).

The covariate *xₚ* (e.g., a predictor) encodes known contextual information regarding position *p* which can include, but is not limited to, information such as trinucleotide context, mappability, segmental duplication, or other information associated with sequence reads. Trinucleotide context can be based on a reference allele and can be assigned numerical (e.g., integer) representation. For instance, "AAA" is assigned 1, "ACA" is assigned 2, "AGA" is assigned 3, etc. Mappability represents a level of uniqueness of the alignment of a read to a particular target region of a genome. For example, mappability is calculated as the inverse of the number of position(s) where the sequence read will uniquely map. Segmental duplications correspond to long nucleic acid sequences (e.g., having a length greater than approximately 1000 base pairs) that are nearly identical (e.g., greater than 90% match) and occur in multiple locations in a genome as a result of natural duplication events (e.g., not associated with cancer or a disease).

The expected mean AD frequency of an SNV at position *p* is modeled by the parameter *µₚ*. In some embodiments, the parameter *µₚ* corresponds to the mean AD count per sequencing depth, *d_{iₚ}.* Because SNV is an example of variant, the parameter *µₚ* can also be referred to as a mean variant frequency. For sake of clarity in this description, the terms *µₚ* and *yₚ* refer to the position-specific sub-models of the Bayesian hierarchical model 225. In some embodiments, *µₚ* is modeled as a Gamma-distributed random variable having shape parameter *α_{zₚ,xₚ}* and rate parameter *β_{zₚ,xₚ}* : ${μ}_{p} ∼ Gamma \left({α}_{{z}_{p} , {x}_{p}}, {β}_{{z}_{p} , {x}_{p}}\right)$ In some embodiments, other functions can be used to represent *µₚ*, examples of which include but are not limited to: a log-normal distribution with log-mean *γ_{zₚ}* and log-standard-deviation *σ_{zₚxₚ}*, a Weibull distribution, a power law, an exponentially-modulated power law, or a mixture of the preceding. The shape parameter *α_{xₚ}* can sometimes be an example of a dispersion parameter *rₚ* in a distribution.

The variance of a distribution can be determined by the mean variant frequency *µₚ* and the dispersion parameter *rₚ*. For example, in the case of a Gamma distribution, the variance, *vₚ*, can be determined by: ${v}_{p} = {λ}_{p} + \frac{{λ}_{p}^{2}}{{r}_{p}}$ Lambda *λₚ* can be the mean variant count, which can be determined by *µₚ* multiplied by sequencing depth, *dᵢₚ.* Also, lambda *λₚ* can be related to the shape parameter *α_{zₚ,xₚ}* and rate parameter *β_{zₚ,xₚ}* by the following: ${λ}_{p} = \frac{{α}_{{z}_{p} , {x}_{p}}}{{β}_{{z}_{p} , {x}_{p}}}$

In the example shown in FIG. 5A, the shape and rate parameters are each dependent on the covariate *xₚ* and the latent variable *zₚ*, though in some embodiments, the dependencies can be different based on various degrees of information pooling during training. For instance, the model can alternately be structured so that *α_{zₚ}* depends on the latent variable but not the covariate. The distribution of AD count of the SNV at position *p* in a human population sample *i* (of a healthy individual) is modeled by the random variable *y_{iₚ}*. The random variable *y_{iₚ}* can also be referred to as a variant count or an observed variant count. In some embodiments, the distribution is a Poisson distribution given a sequencing depth *dᵢₚ* of the sample at the position: $\left.{y}_{{i}_{p}}\right| {d}_{{i}_{p}} ∼ Poisson \left({d}_{{i}_{p}}⋅{μ}_{p}\right)$ In some embodiments, other functions can be used to represent *y_{iₚ}*, examples of which include but are not limited to: negative binomial, Conway-Maxwell-Poisson distribution, zeta distribution, and zero-inflated Poisson. For example, the random variable *y_{iₚ}* can be modeled by a negative binomial distribution: $\left.{y}_{{i}_{p}}\right| {d}_{{i}_{p}} ∼ NB \left({d}_{{i}_{p}}⋅{μ}_{p}, {r}_{p}\right)$

The mean variant frequency *µₚ*, the mean variant count *λₚ* (*λₚ* = *d_{iₚ}* · *µₚ*), and the dispersion parameter *rₚ* can be referred to noise parameters because those parameters affect the distribution of the random variable of variant count *y_{iₚ}*.

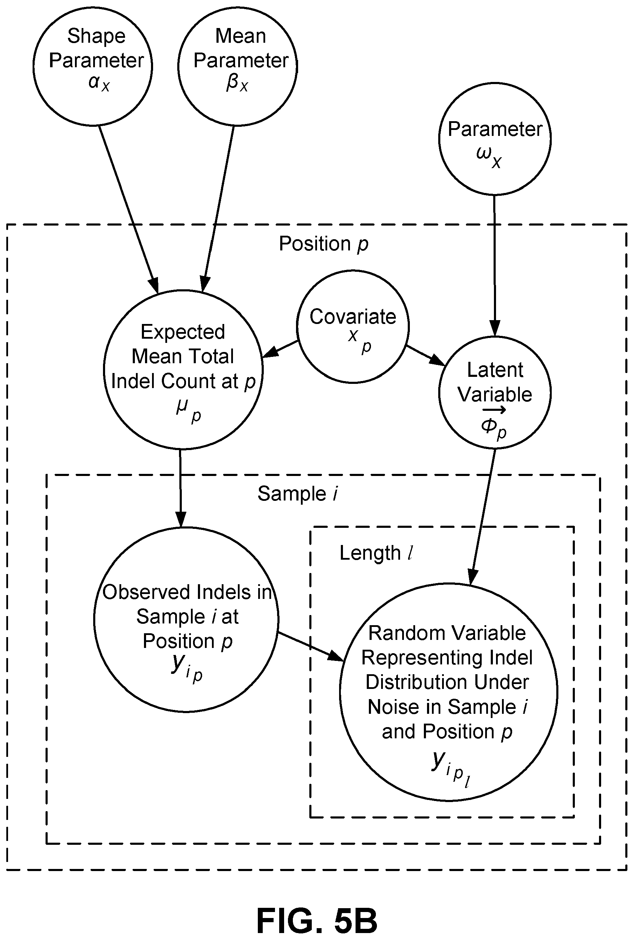

FIG. 5B shows dependencies between parameters and sub-models of a Bayesian hierarchical model for determining true insertions or deletions according to various embodiments. In contrast to the SNV model shown in FIG. 5A, the model for indels shown in FIG. 5B includes different levels of hierarchy. The covariate *xₚ* encodes known features at position *p* and can include, e.g., a distance to a homopolymer, distance to a RepeatMasker repeat, or other information associated with previously observed sequence reads. Latent variable *̅ϕ̅ₚ̅*̅ can be modeled by a Dirichlet distribution based on parameters of the vector *ω̅ₓ,* which represent indel length distributions at a position and can be based on the covariate. In some embodiments, *ω̅ₓ* is also shared across positions (*ω̅_{x,p}* ) that share the same covariate value(s). Thus, for example, the latent variable can represent information such as that homopolymer indels occur at positions 1, 2, 3, etc. base pairs from the anchor position, while trinucleotide indels occur at positions 3, 6, 9, etc. from the anchor position.

The expected mean total indel frequency at position *p* is modeled by the distribution *µₚ*. In some embodiments, the parameter *µₚ* corresponds to the mean indel count per sequencing depth, *dᵢₚ.* Because indel is an example of variant, the parameter *µₚ* can also be referred to as a mean variant frequency. In some embodiments, the distribution is based on the covariate and has a Gamma distribution having a shape parameter *α_{xₚ}* and a rate parameter *β_{xₚzₚ}*: ${μ}_{p} ∼ Gamma \left({α}_{{x}_{p}}, {β}_{{x}_{p} {z}_{p}}\right)$ In some embodiments, other functions can be used to represent *µₚ*, examples of which include but are not limited to: negative binomial, Conway-Maxwell-Poisson distribution, zeta distribution, and zero-inflated Poisson. The shape parameter *α_{xₚ}* can sometimes be an example of a dispersion parameter *rₚ* in a distribution.

The variance of a distribution can be determined by the mean variant frequency *µₚ* and the dispersion parameter *rₚ*. For example, in the case of a Gamma distribution, the variance, *vₚ*, can be determined by: ${v}_{p} = {λ}_{p} + \frac{{λ}_{p}^{2}}{{r}_{p}}$

Lambda *λₚ* can be the mean variant count, which can be determined by *µₚ* multiplied by sequencing depth, *dᵢₚ.* Also, lambda *λₚ* can be related to the shape parameter *α_{zₚ,xₚ}* and rate parameter *β_{zₚ,xₚ}* by the following: ${λ}_{p} = \frac{{α}_{{z}_{p} , {x}_{p}}}{{β}_{{z}_{p} , {x}_{p}}}$

The observed indels at position *p* in a human population sample *i* (of a healthy individual) is modeled by the distribution *y_{iₚ}*. The random variable *y_{iₚ}* can also be referred to as a variant count or an observed variant count. Similar to the example in FIG. 5A, in some embodiments, the distribution of indel intensity is a Poisson distribution given a sequencing depth *d_{iₚ}* of the sample at the position: $\left.{y}_{{i}_{p}}\right| {d}_{{i}_{p}} ∼ Poisson \left({d}_{{i}_{p}}⋅{μ}_{p}\right)$ In some embodiments, other functions can be used to represent *y_{iₚ}*, examples of which include but are not limited to: negative binomial, Conway-Maxwell-Poisson distribution, zeta distribution, and zero-inflated Poisson. For example, in some examples, the random variable *y_{iₚ}* is modeled by a negative binomial distribution: $\left.{y}_{{i}_{p}}\right| {d}_{{i}_{p}} ∼ NB \left({d}_{{i}_{p}}⋅{μ}_{p}, {r}_{p}\right)$

The mean variant frequency *µₚ*, the mean variant count *λₚ* (*λₚ* = *d_{iₚ}* · *µₚ*), and the dispersion parameter *rₚ* can be referred to noise parameters because those parameters affect the distribution of the random variable of variant count *y_{iₚ}*.

Because indels can be of varying lengths, an additional length parameter is present in the indel model that is not present in the model for SNVs. As a consequence, the example model shown in FIG. 5B has an additional hierarchical level (e.g., another sub-model), which is again not present in the SNV models discussed above. The observed count of indels of length *l* (e.g., up to 100 or more base pairs of insertion or deletion) at position *p* in sample i is modeled by the random variable ${y}_{{i}_{{p}_{l}}}$, which represents the indel distribution under noise conditional on parameters. The distribution can be a multinomial given indel intensity *y_{iₚ}* of the sample and the distribution of indel lengths *̅ϕ̅ₚ̅*̅ at the position: ${\vec{y}}_{{i}_{{p}_{l}}} \left|{y}_{{i}_{p}}, \vec{{ϕ}_{p}}\right. ∼ Multinom \left({y}_{{i}_{p}}, \vec{{ϕ}_{p}}\right)$ In some embodiments, a Dirichlet-Multinomial function or other types of models can be used to represent ${y}_{{i}_{{p}_{l}}}$.

By architecting the model in this manner, the machine learning engine 220 can decouple the learning of indel intensity (i.e., noise rate) from learning of indel length distribution. Independently determining inferences for an expectation for whether an indel will occur in healthy samples and expectation for the length of the indel at a position may improve the sensitivity of the model. For example, the length distribution can be more stable relative to the indel intensity at a number of positions or regions in the genome, or vice versa.

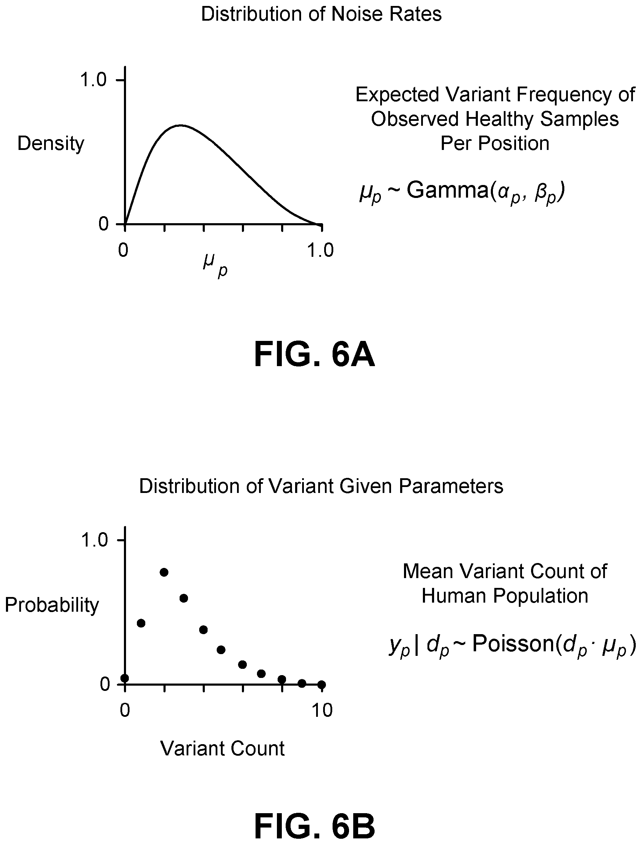

FIGS. 6A-B illustrate diagrams associated with a Bayesian hierarchical model 225 according to various embodiments. The graph shown in FIG. 6A depicts the distribution *µₚ* of noise rates, i.e., likelihood (or intensity) of SNVs or indels for a given position as characterized by a model. The continuous distribution represents the mean variant frequency *µₚ* of non-cancer or non-disease mutations (e.g., mutations naturally occurring in healthy tissue) based on training data of observed healthy samples from healthy individuals (e.g., retrieved from the sequence database 210). Though not shown in FIG. 6A, in some embodiments, the shape and rate parameters can be based on other variables such as the covariate *xₚ* or latent variable *zₚ*. The graph shown in FIG. 6B depicts the distribution of AD at a given position for a sample of a subject, given parameters of the sample such as sequencing depth *dₚ* at the given position. The discrete probabilities for a draw of *µₚ* are determined based on the predicted true mean AD count of the human population based on the expected mean distribution *µₚ*.

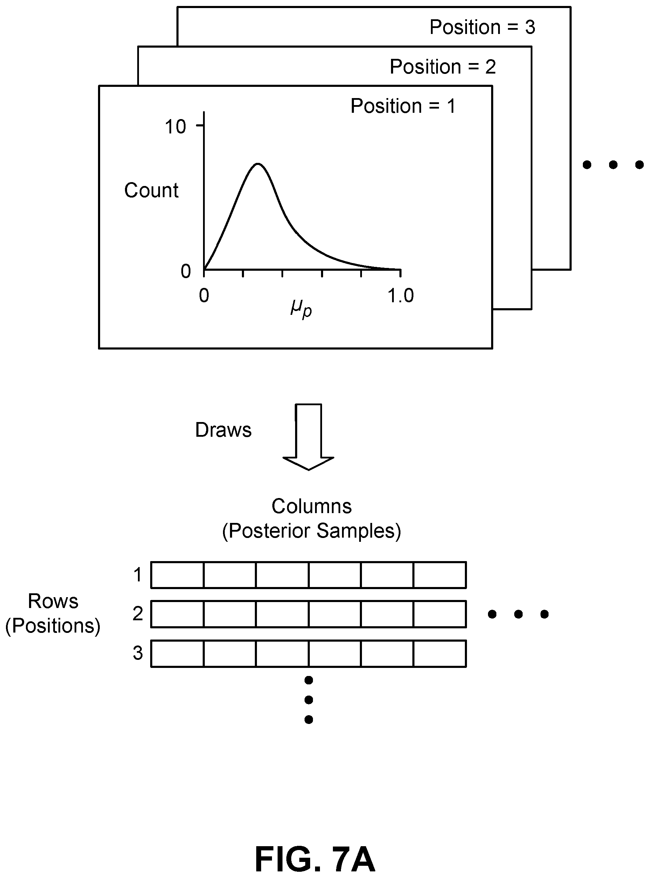

FIG. 7A is a diagram of an example process for determining parameters by fitting a Bayesian hierarchical model 225 according to various embodiments. To train a model, the machine learning engine 220 samples iteratively from a posterior distribution of expected noise rates (e.g., the graph shown in FIG. 6B) for each position of a set of positions. The machine learning engine 220 can use Markov chain Monte Carlo (MCMC) methods for sampling, e.g., a Metropolis-Hastings (MH) algorithm, custom MH algorithms, Gibbs sampling algorithm, Hamiltonian mechanics-based sampling, random sampling, among other sampling algorithms. During Bayesian Inference training, parameters are drawn from the joint posterior distribution to iteratively update all (or some) parameters and latent variables of the model (e.g., *̅θ̅*̅, *zₚ*, *α_{zₚ,xₚ}*, *β_{zₚ,xₚ}*, *µₚ,* etc.).

In some embodiments, the machine learning engine 220 performs model fitting by storing draws of *µₚ* in the parameters database 230. The model is trained or fitted through posterior sampling, as previously described. In some examples, the draws of *µₚ* are stored in a matrix data structure having a row per position of the set of positions sampled and a column per draw from the joint posterior (e.g., of all parameters conditional on the observed data). The number of rows R can be greater than 6 million and the number of columns for N iterations of samples can be in the thousands. In some embodiments, the row and column designations are different than the embodiment shown in FIG. 7A, e.g., each row represents a draw from a posterior sample, and each column represents a sampled position (e.g., transpose of the matrix example shown in FIG. 7A).

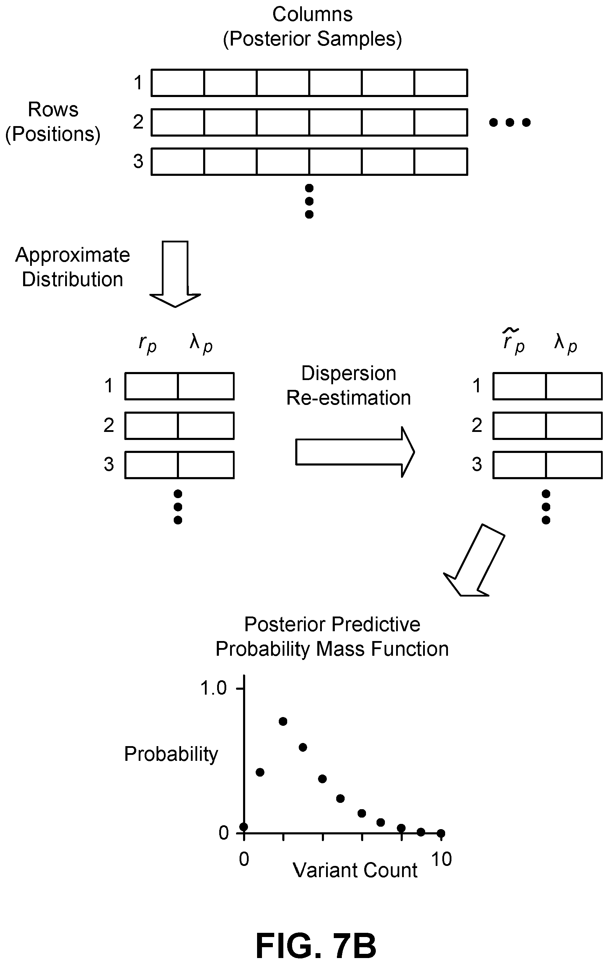

FIG. 7B is a diagram of using parameters from a Bayesian hierarchical model 225 to determine a likelihood of a false positive according to various embodiments. The machine learning engine 220 can reduce the R rows-by-N column matrix shown in FIG. 7A into an R rows-by-2 column matrix illustrated in FIG. 7B. In some examples, the machine learning engine 220 determines various noise parameters such as a dispersion parameter *rₚ* (e.g., shape parameter) and mean parameter *λₚ* (which can also be referred to as a mean rate parameter *λₚ*) per position across the posterior samples *µₚ*. The dispersion parameter *rₚ* can be determined as ${r}_{p} = \frac{{{λ}_{p}}^{2}}{{{v}_{p}}^{2}}$, where *λₚ* and *vₚ* are the mean and variance of the sampled values of *µₚ* at the position, respectively. Those of skill in the art will appreciate that other functions for determining *rₚ* can also be used such as a maximum likelihood estimate. Different noise parameters can be determined for different read tiers. For example, each read tier can have different values of *λₚ* and *rₚ*.

The machine learning engine 220 can also perform dispersion re-estimation of the dispersion parameters in the reduced matrix, given the rate parameters. In some embodiments, following Bayesian training and posterior approximation, the machine learning engine 220 performs dispersion re-estimation by retraining for the dispersion parameters ${{r}_{p}}^{˜}$ based on a negative binomial maximum likelihood estimator per position. The rate parameter can remain fixed during retraining. In some embodiments, the machine learning engine 220 determines the dispersion parameters *r'ₚ* at each position for the original AD counts of the training data (e.g., *y_{iₚ}* and *d_{iₚ}* based on reference samples, stratified by read tier). The machine learning engine 220 determines ${{r}_{p}}^{˜} = max \left({r}_{p}, r{′}_{p}\right)$, and stores ${{r}_{p}}^{˜}$ in the reduced matrix. Those of skill in the art will appreciate that other functions for determining ${{r}_{p}}^{˜}$ can also be used, such as a method of moments estimator, posterior mean, or posterior mode.

During the application of trained models, the processing system 200 can access the dispersion (e.g., shape) parameters ${{r}_{p}}^{˜}$ and rate parameters *λₚ* to determine a function parameterized by ${{r}_{p}}^{˜}$ and *λₚ*. The function can be used to determine a posterior predictive probability mass function (or probability density function) for a new sample of a subject. Based on the predicted probability of a certain AD count at a given position, the processing system 200 can account for site-specific noise rates per position of sequence reads when detecting true positives from samples. Referring back to the example use case described with respect to FIG. 4, the PMFs shown for Mutations A and B can be determined using the parameters from the reduced matrix of FIG. 7B. The posterior predictive probability mass functions can be used to determine the probability of samples for Mutations A or B having an AD count at certain position.

The Bayesian hierarchical models and distributions used to model various parameters in the Bayesian hierarchical models can be trained separately for different read tiers of variant reads. For example, each read tier can have its own Bayesian hierarchical model that has its own parameters such as *α_{xₚ}*, *β_{xₚ}*, *µₚ*, etc.

For more information regarding the training and use of the Bayesian hierarchical models that model the noise levels of a sequencing dataset, reference is made to U.S. Patent Application No. 16/153,593, filed on October 5, 2018 and entitled "Site-Specific Noise model for Targeted Sequencing,".

### V. EXAMPLE READ TIERS

FIG. 8A includes diagrams illustrating different categories or read tiers of sequence reads, according to various embodiments. As contemplated herein, sequence reads can be associated with different read tiers representing different quality levels of the reads, whereby the quality levels can be based on a variant location relative to overlapping segments of the sequence reads. A higher-quality read tier corresponds to a lower noise level or otherwise a lower error rate, and a lower quality read tier corresponds to a higher noise level or otherwise a higher error rate.

It is noted that in a sequence amplification process (e.g., massively parallel sequencing), one or more sequences of a sample (e.g., an individual) can be cleaved into different fragments in a pseudo-random fashion. In some cases, not all fragments are ligated with UMIs such that some of the fragments are washed away before the ligated fragments are enriched. Hence, the enriched fragments are at least partially random in each sequencing run. The extent of overlap between different fragments can vary. For example, some of the enriched fragments can have overlapping regions that can be stitched with other enriched fragments. Some enriched fragments can also have complementary sequences (e.g., forward and reverse sequences, positive and negative sequences, top and bottom sequences, 5' to 3' and 3' to 5' sequences) that are enriched, thus generating a double-stranded read for all or part of the entire sequence read. As a result, variant reads at different sequence locations can, in some examples, include a complementary and/or overlapping sequence read to confirm the variant. Hence, each variant read can correspond to a different read tier quality. For example, a variant read at a location in which both complementary strands of fragments are enriched often has better quality than another variant read at a second location in which only a single fragment is enriched. There is an increased likelihood that a variant read at a location that is not included within an overlapping region or a complementary region is attributable to noise, and not to an actual variant presented in the sample of the subject.

FIG. 8A illustrates four different examples of read tiers. In some embodiments, the sequence reads are separated into the read tiers based on a potential variant location of interest within the sequence reads relative to overlapping and complementary locations within the sequence reads. In other words, a sequence read is classified into one of the four read tiers based on whether the potential variant location read is included or otherwise fully embedded within an overlapping region (i.e., a stitched region) and whether the variant location is included or otherwise fully embedded within into a complementary region (i.e., a double-stranded region, duplex region).

By way of example, in FIG. 8A, the potential variant locations are shaded. A first example read tier 810 includes variant reads that fall with both double-stranded (also referred to as "duplex" or (complementary") and stitched sequence reads. For instance, at least two 5' to 3' sequence reads have an overlapping region and can be stitched together. Similarly, at least two 3' to 5' sequence reads have an overlapping region and can also be stitched together. In the example first read tier 810, the potential variant location is located or fully embedded within the overlapping or otherwise stitched regions, and thus the sequence reads include a stitched region. Likewise, at least a portion of the 5' to 3' sequence reads and a portion of the 3' to 5' sequence reads are complementary to each other, and the potential variant location is located within the complementary region (e.g., the potential variant location is fully embedded within both top and bottom sequence reads at their common region of overlap). Accordingly, in addition to including a stitched region, the sequence reads include a double-stranded region, and the potential variant read belongs to the first read tier 810 representing double-stranded and stitched reads.

At FIG. 8A, a second example read tier 820 includes variant reads located within portions of double-stranded but unstitched sequence reads. In the second read tier 820, a portion of the 5' to 3' sequence reads and a portion of the 3' to 5' sequence read are complementary to each other, and the potential variant location is located within the complementary region. Hence, the sequence reads include a double-stranded region. However, the potential variant location is not included within any of the overlapping regions or otherwise stitched regions of sequence reads. Specifically, this example stratification is despite the fact that the two 5' to 3' sequence reads can be stitched together, because the potential variant location is not included within the overlapping regions or otherwise stitchable regions. Accordingly, while the sequence reads include a double-stranded region, the sequences reads do not include a stitched region, and the potential variant read belongs to the second read tier 820 representing double-stranded but unstitched reads.

A third example read tier 830 includes variant reads located or otherwise fully embedded within single-stranded (e.g., non-duplex) and stitched reads. In the third read tier 830, the potential variant location is included within the overlapping region of two or more sequence reads, and thus the sequence reads include a stitched region. However, the sequence reads are single-stranded because the sequence reads (such as the two illustrated 5' to 3' sequence reads) do not include a complementary region (e.g., the sequence reads are based on the 5' to 3' strands only and are not supported by a complementary 3' to 5' strand). In some cases (not illustrated), one or more complementary sequence reads (e.g., a 3' to 5' sequence read) can be found at example read tier 3 but not include the potential variant location. Thus, the potential variant read belongs to the third read tier 830 representing single-stranded but stitched reads.

Further as shown at FIG. 8A, a fourth example read tier 840 includes variant reads located at single-stranded and unstitched reads. Like the third read tier 830, the fourth read tier 840 represents single-stranded reads because the illustrated sequence reads do not include a complementary region containing the variant location (or in some cases (not illustrated), do further include a complementary region, but the potential variant location is not located or fully embedded within the complementary region). Thus, the fourth read tier 840 represents unstitched reads because the potential variant location is not included within an overlapping region of two sequence reads.

In some embodiments, sequence reads of a sample can be stratified into the four read tiers illustrated in FIG. 8A. In some embodiments, there can be an additional fifth read tier that corresponds to the lowest quality of variant reads, such as single-stranded and unstitched sequence reads that include potential variant locations near an end of one or more sequence reads. For example, if a single-stranded and unstitched sequence read includes a potential variant location is within a predetermined threshold number of bases (e.g., within about 7 bases or within about 30 bases) from either end of the sequence read, the sequence read can be classified into the fifth read tier. In some embodiments, each of the four read tiers shown in FIG. 8A can be sub-divided into two sub-tiers: a first, lower-quality sub-tiers corresponding to sequence reads including potential variant locations near either end of one or more sequence reads and a second higher-quality sub-tiers including potential variant locations more than a threshold distance from the ends of one or more sequence reads.

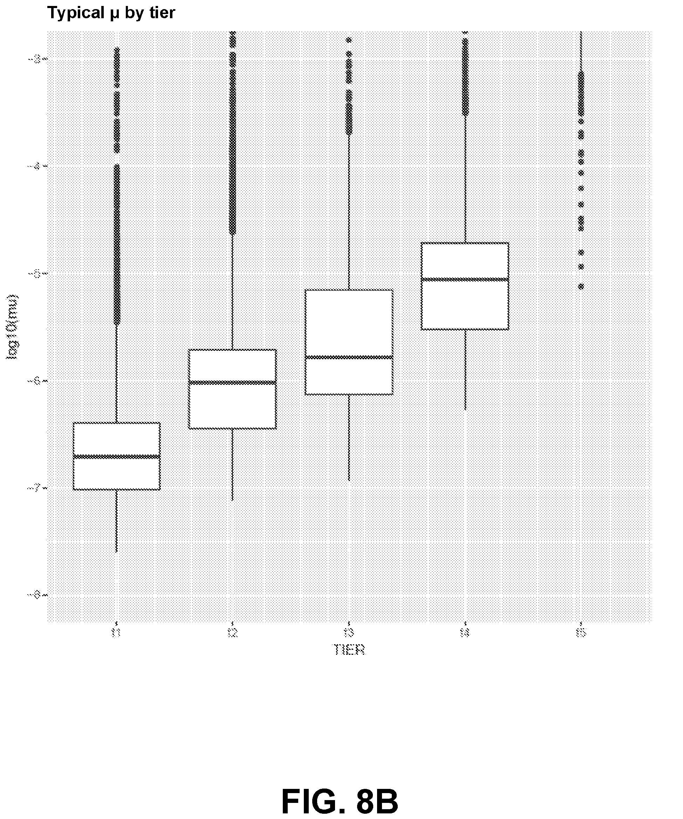

FIG. 8B shows an experimental result that illustrates different qualities of the read tiers of FIG. 8A, according to various embodiments. A higher quality of read tier corresponds to a lower error rate and/or lower noise level. In other words, a variant read (e.g., a detected SNV or indel at the potential variant location) in a sequence read that is stratified to a high-quality read tier is more likely attributable to an actual mutation of the sample as opposed to a random event (e.g., due to noise) than a variant read in a sequence read that is stratified to a lower-quality read tier. FIG. 8B is a plot of log 10 of mean error rates of reference samples (e.g., healthy individuals) for different read tiers t1-t5. Tier 1 (t1) refers to the first read tier 810 in FIG. 8A, tier 2 (t2) refers to the second read tier 820, etc. Tier 5 (t5) refers to the single-stranded and unstitched read tier and/or potential variant locations that are located near either end (e.g., within 7 bases from an end) of a sequence read. For tier 1, log base 10 of its mean error rate mu is at about -6.3 to -7. In other words, there is an average error rate of about a 10^{-6.3} to 10⁻⁷ variant read per sequencing depth for a healthy individual. On the other hand, for tier 4, log base 10 of its mean error rate mu is at about -4.7 to -5.5. In some aspects, FIG. 8B generally shows that the mean error rate increases across tiers 1-4, from a tier 1 mean error rate of about 1/1,000,000 - 1/10,000,000, to a tier 2 mean error rate closer to about 1/1,000,000, increasing again in a tier 3 mean error rate of about < 1/1,000,000, and still increasing again to a tier 4 error rate of about 1/100,000. Thus, a variant read detected at tier 4 is about 100 times more likely to be an error allele than a variant read detected at tier 1. In other words, the fourth read tier is relatively noisier and more error-prone than the first read tier. In other words, the fourth read tier is relatively noisier and more error-prone than the first read tier. Further, as shown for example tier 5, there is no mean error rate mu (or otherwise no meaningful mean error rate) due to its sequence reads being at such low quality that they are insignificant or otherwise discarded from the analysis.

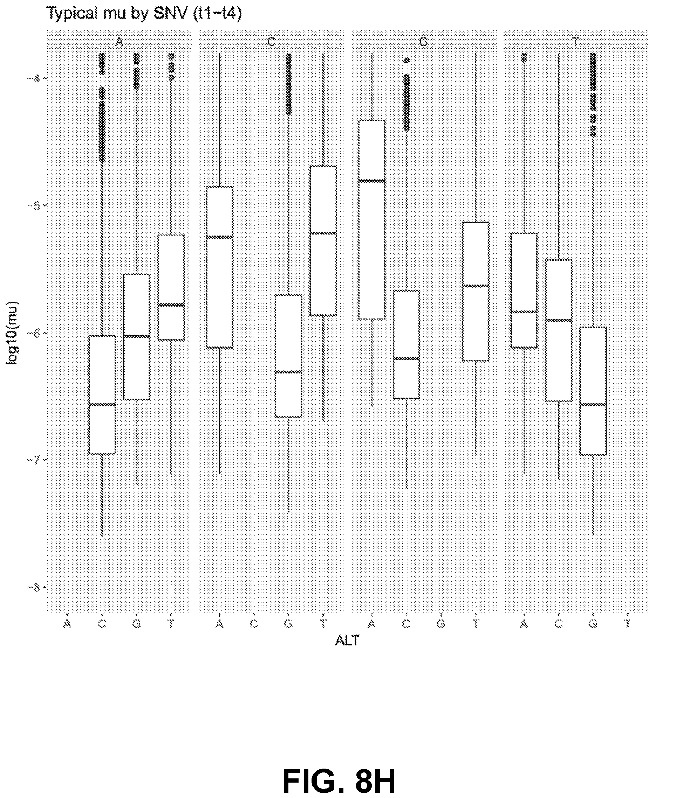

Sequence reads can, additionally or alternatively, be classified into different read tiers by other classification methods. For example, if the variants are SNVs, each read tier can be further sub-divided into twelve additional sub-tiers based on the types of nucleotide substitution (e.g., A>C, A>T, G>C, etc.) (see, e.g., FIG. 8H discussed below). Because there are four nucleotides and each nucleotide is substituted by a different nucleotide in an SNV, there are a total of twelve different types of SNVs.

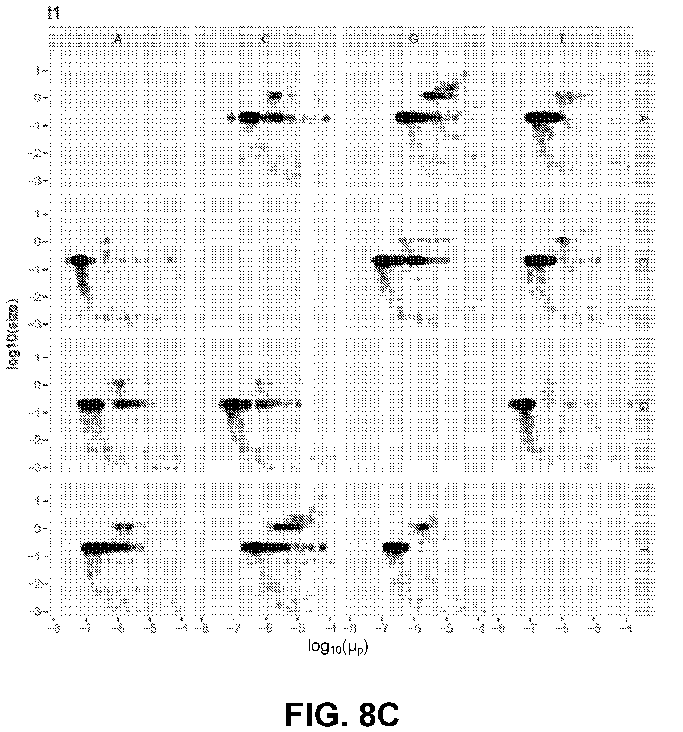

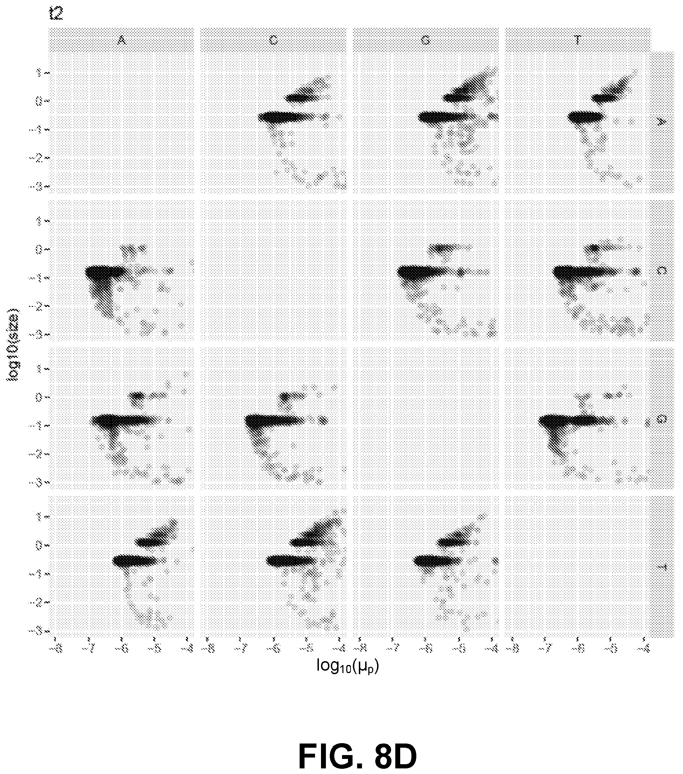

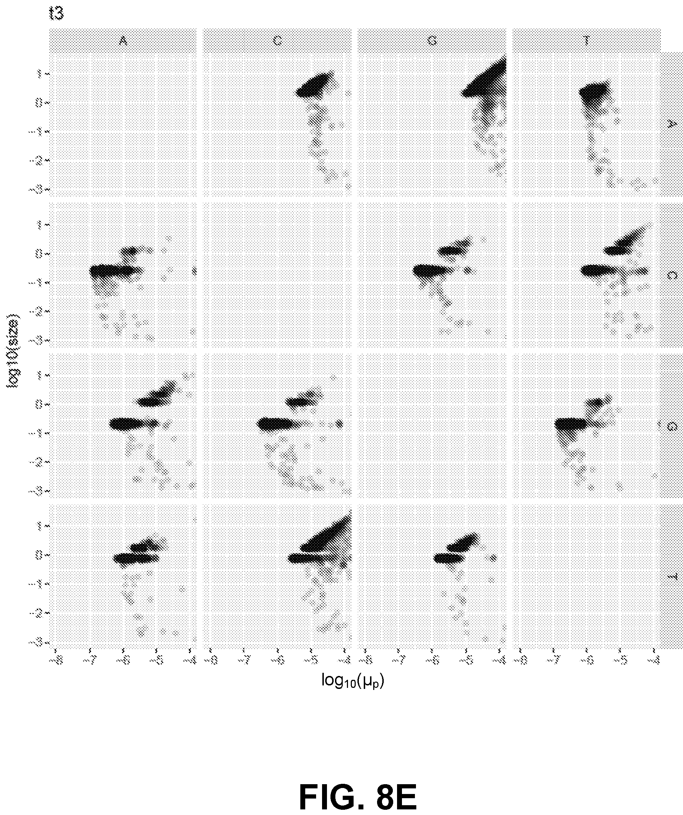

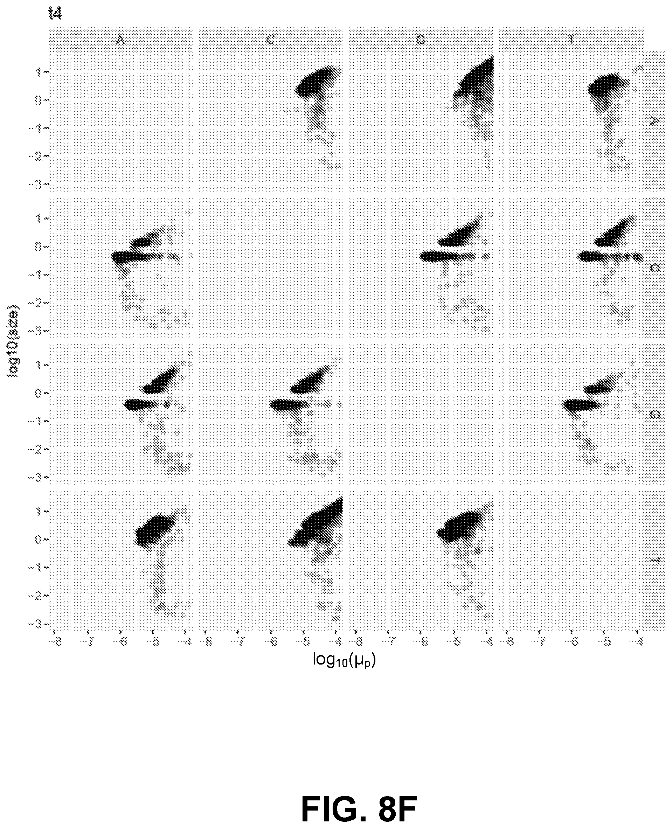

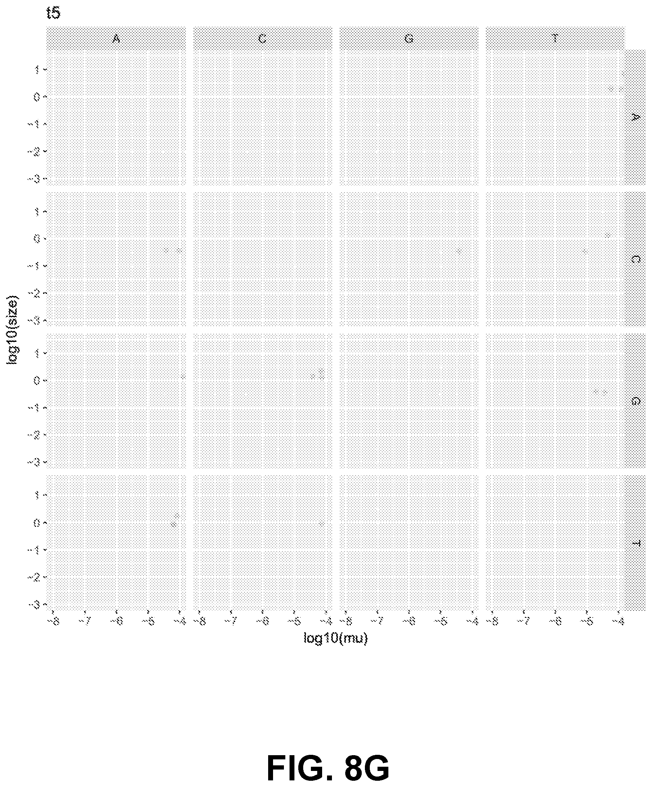

FIGS. 8C through 8G show experimental results of the read tiers of FIG. 8A when the sequence reads are first stratified into read tiers by the manner described in FIG. 8A and are further stratified into twelve sub-read tiers based on the types of nucleotide substitution. Specifically, FIGS. 8C-8G illustrate mean error rate mu (*µ*) and size parameters for a statistical model (e.g., a negative binomial) for the error distribution of alternate reads at a certain position, such that the error distribution at each position (e.g. each point) is conditional on actual read depths seen in a given sample. The model is stratified by different categories of reads (e.g., tiers), such that FIG. 8C illustrates the results of different types of nucleotide substitution for the first read tier (i.e., double-stranded and stitched reads), FIG. 8D illustrates the results of different types of nucleotide substitution for the second read tier (i.e., double-stranded but unstitched reads), FIG. 8E illustrates the results of different types of nucleotide substitution for the third read tier (i.e., single-stranded and stitched reads), and FIG. 8F illustrates the results of different types of nucleotide substitution for the fourth read tier. FIG. 8G illustrates results from the fifth read tier corresponding to the lowest quality reads and high error rates that exceed the axes shown. It is noted that for FIGS. 8C-8G, nucleotide bases A, C, G, and T horizontal across the top of the plots refer to alternate bases, while nucleotide bases A, C, G, and T vertical along the right side of the plots refer to reference bases.

Referring to FIG. 8C, the twelve different distributions of typical variant frequency in different SNVs show that the first read tier can be further divided into twelve sub-tiers to further improve noise models. The row corresponds to the original nucleotide while the columns correspond to the changed nucleotide. For example, the cell of the third row and first column can correspond to an SNV of G to A. The experiment shows that a sub-read tier for C to A (i.e., second row, first column), whose distribution of *µ* concentrates in the range of -7 to -8 in a base 10 logarithmic scale, is likely to be less noisy than a sub-read tier for T to C (i.e., fourth row, second column), whose distribution of *µ* spreads across in the range of -5 to -7 in a base 10 logarithmic scale.

Comparing the differences among FIG. 8C through FIG. 8G, the distributions of mean error rate *µ* in a logarithmic scale show a general trend of shifting towards zero (i.e., *µ* becoming larger) and eventually exceeding zero (i.e., at FIG. 8G) as the read tier changes from the first read tier to the fifth read tier. For example, focusing on the sub-read tier for T to G (i.e., fourth row, third column), the distribution of *µ* in logarithmic scale shifts from between -6 and -7 in the first read tier to between -4 and -5 in the fourth read tier. Thus, FIG. 8C through FIG. 8G demonstrate that as the read tiers become noisier, mean error rates *µ* become higher.

Turning now to FIG. 8H, an experimental result is shown illustrating different mean error rates *µ* at specific SNV nucleotide substitutions taken across the read tiers t1-t4 of FIG. 8A, according to various embodiments described herein. Specifically, FIG. 8B is a plot of log 10 of mean error rates *µ* of reference samples (e.g., healthy individuals) in a logarithmic scale for different SNVs observed across the read tiers t1-t4. It is noted that the statistical model for error distribution described herein can be stratified by different categories of reads (tiers) and/or further by different SNVs as shown at FIG. 8H.

### VI. EXAMPLE DATA PROCESSING WITH STRATIFIED READS

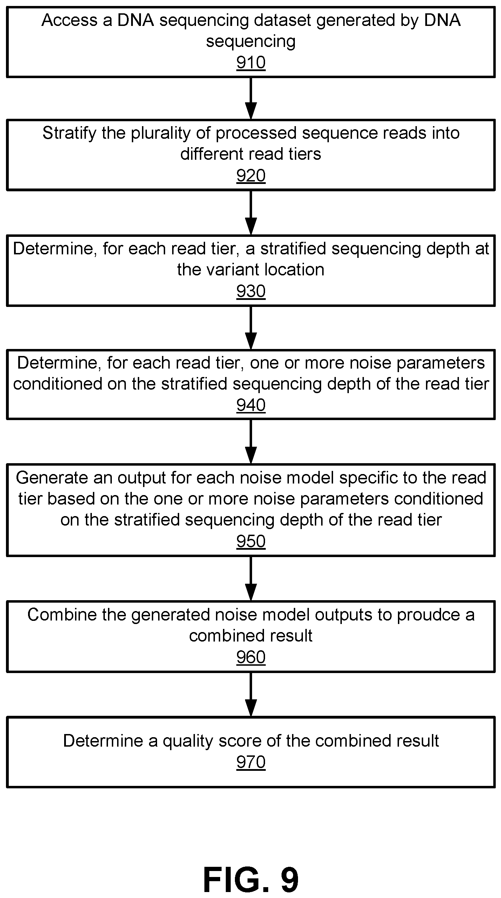

FIG. 9 is a flowchart depicting a process for analyzing the DNA dataset of a sample using stratified noise models, according to some embodiments. The process can be used to process a DNA sequencing dataset of a sample such as an individual's sample including cfDNA to generate a quality score representing a likelihood of the individual having a variant at a potential variant location. The higher the quality score determined by the process, the more likely that the variant reads are results from an actual mutation instead of noise.

In step 910, a processing system can access a DNA sequencing dataset generated by DNA sequencing. For example, the DNA sequencing can be a type of massively parallel DNA sequencing, such as next-generation sequencing (NGS). The DNA sequencing dataset includes a plurality of processed sequence reads that include a variant location of interest (e.g., a specific gene location in a DNA sequence). At least some of the processed sequence reads can be generated from collapsing and stitching of raw sequence reads in the DNA sequencing, generated such as by the process described in FIG. 3. For example, a typical run of an NGS could generate millions or even billions of sequence reads. Some of the raw sequence reads can be included in a genetic locus that includes the variant location of interest. The raw sequence reads can, in turn, be processed by collapsing and stitching to generate the processed sequence reads. It is noted that while DNA sequencing is described in the present example, RNA sequencing can also be implemented for analysis herein.

The processed sequence reads that include the variant location of interest can be of different base-pair lengths and of different extent of overlapping and/or complementing. In step 920, the processing system can stratify the plurality of processed sequence reads into different read tiers. The different read tiers can be stratified based on the quality of the sequence reads. For example, the processed sequence reads can be stratified based on whether the variant location is included in an overlapping region and/or is included in a complementary region, as discussed in association with FIG. 8A. Other ways to stratify the processed sequence reads are also possible. For example, the processed sequence reads can also be stratified based on the type of nucleotide substitution, whether the variant location is near the end of a sequence, etc. In some embodiments, the different read tiers include at least four read tiers. In some examples, the four read tiers are (1) a double-stranded, stitched read tier, (2) a double-stranded, unstitched read tier, (3) a single-stranded, stitched read tier, and (4) a single-stranded, unstitched read tier.

In step 930, the processing system can determine, for each read tier, a stratified sequencing depth at the variant location. For each read tier, the stratified sequencing depth can be the sequencing depth of the sequence reads that are stratified into the read tier. In other words, a stratified sequencing depth can be the total number of sequence reads that are stratified into the read tier. The processing system can also determine the actual variant count for each read tier. For example, for a read tier, a majority of the sequence reads may not contain an actual variant (whether it is an SNV or indel) at the variant location. In some cases, only a handful of sequence reads include an actual variant at the variant location. A stratified variant count can be the total number of actual variant counts for a particular read tier.

In step 940, the processing system can determine, for each read tier, one or more noise parameters conditioned on the stratified sequencing depth of the read tier. The noise parameters can be the parameters of a noise model that is specific to the read tier. For example, the processing system can include a plurality of stratified noise models, each specific to a read tier. The stratified noise models (or some of them) can correspond to the Bayesian hierarchical models described in FIG. 5A through FIG. 7B. In other words, in some embodiments, each read tier has its own Bayesian hierarchical model. Each noise model of a read tier can be trained using different training sets of DNA sequencing samples. By way of example, DNA sequencing datasets of a plurality of reference individuals, such as healthy individuals, can be collected. The processed sequence reads of the datasets of the reference individuals can be stratified by read tiers. The stratified processed sequence reads for each read tier can be used as the stratified training set of DNA sequencing samples to train the stratified noise model for the read tier. Various distributions of a stratified noise model, such as the Gamma distribution and the Poisson distribution discussed in association with FIGS. 5A and 5B, can be determined based on the stratified training set.

A probability distribution of a stratified variant count for each read tier can be modeled by a noise distribution. The probability distribution of the stratified variant count can depend on the type of distribution used and one or more parameters that define the noise distribution. For example, in the case of a Bayesian hierarchical model discussed, the distribution of the stratified variant count can correspond to a posterior distribution conditioned on two parameters. The parameters can be the stratified mean variant count conditioned on the stratified sequencing depth and the dispersion parameter. Each of the parameters can further correspond to one or more prior distributions that affect the parameters. For example, the stratified mean variant count conditioned on the stratified sequencing depth can be modeled by a Gamma distribution. Because a prior distribution can describe the distribution of a parameter, the prior distribution can also be referred to as a parameter distribution.

For each read tier, the processing system can determine the one or more noise parameters conditioned on the stratified sequencing depth by inputting the stratified sequencing depth, obtained from the dataset of the subject, to the trained noise model. For example, a trained noise model can access a parameter distribution (e.g., a prior distribution) specific to the read tier. The parameter distribution can be formed based on a stratified training set of reference individuals and can describe the distribution of the stratified training set. The trained noise model can use the parameter distribution to determine the noise parameter conditioned on the stratified sequencing depth corresponding to the read tier.

While a Bayesian hierarchical model is used as an example of a noise model, in various embodiments different types of trained machine learning models can be used as the noise models. Also, depending on the model used, different noise parameters can be used to model the noise distribution.

In step 950, the processing system can generate an output for the noise model specific to a read tier based on the one or more noise parameters conditioned on the stratified sequencing depth of the read tier. The generation of the output can be repeated for different read tiers. Depending on the embodiments, different types of outputs can be generated. For instance, in some embodiments, each stratified noise model does not perform further computation after the noise parameters are determined. The output of a noise model can be the one or more noise parameters that are conditioned on the stratified sequencing depth determined for each tier. In the case of a negative binomial distribution being used to as a noise distribution to model the stratified variant count, the output of the noise model can be a stratified mean variant count conditioned on the stratified sequencing depth and a dispersion parameter. In some embodiments, after determining the noise parameters, each stratified noise model can generate a posterior distribution. In such an embodiment, the output of a noise model specific to a read tier can be a likelihood that a variant count of the read tier for subsequently observed data being greater than or equal to a total variant count observed in the DNA dataset of the subject individual is attributable to noise. Other suitable outputs are also possible.

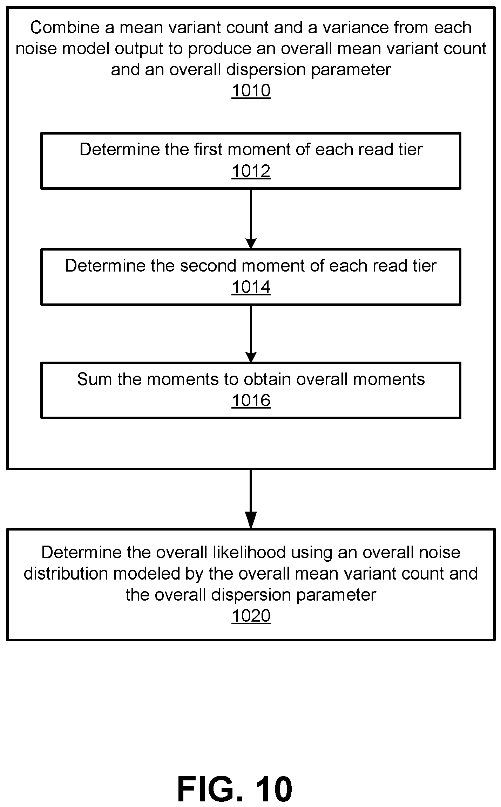

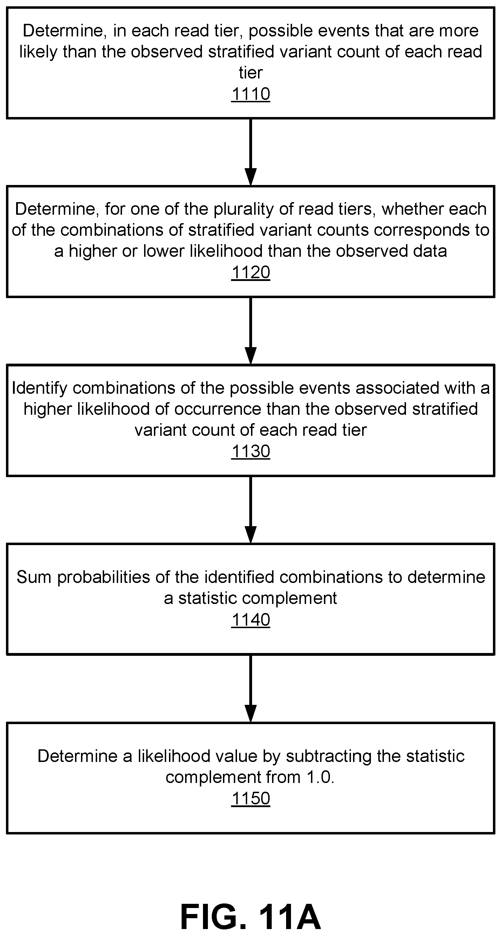

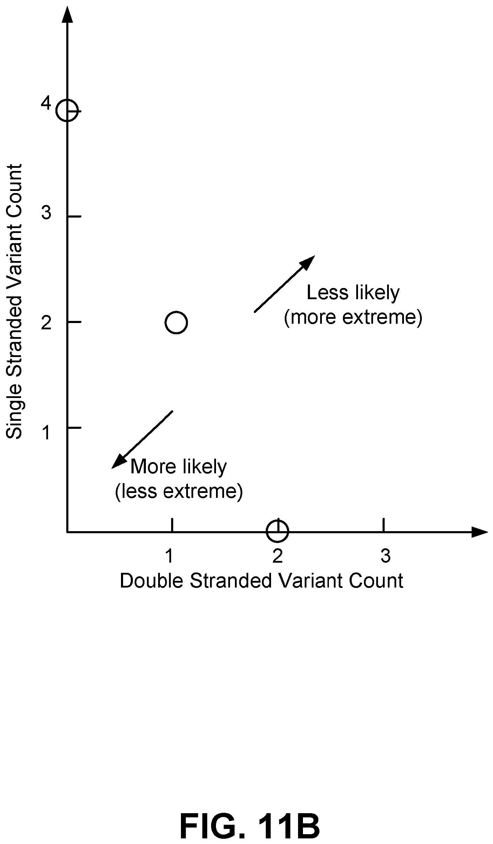

In step 960, the processing system can combine the generated noise model outputs to produce a combined result. The combined result can be a representation of the overall processing result of the DNA sequencing dataset of the subject individual. The combined result can take any suitable forms. In some embodiments, the combined result can include a likelihood that a total variant count for subsequently observed data being greater than or equal to a total variant count observed in the plurality of processed sequence reads is attributable to noise. Put differently, the likelihood can represent the likelihood that an event would be as or more extreme than a total variant count observed in the plurality of processed sequence reads of the DNA dataset of the subject individual. In some cases, the likelihood can correspond to the p-value that is used in a null hypothesis. How the outputs of the stratified noise models can be combined to generate a combined result can depend on different embodiments. In some embodiments, a moment matching method, which will be discussed in detail in FIG. 10, can be used. In some embodiments, an integration method (e.g., a summing method), which will be discussed in detail in FIGS. 11A and 11B, can be used.

In step 970, the processing system can determine a quality score of the combined result. In some examples, the combined result, such as in the form of likelihood P (e.g., a p-value), can be converted into a Phred-scaled quality score, where *Q* = -10 · *log*₁₀ *P* . For example, a Phred quality score of 20 indicates a P = 1/100 chance of an incorrect variant call, and a Phred quality score of 60 indicates a P = 1/1,000,000 chance of an incorrect variant call. Thus, a higher Phred quality score corresponds to a greater confidence for the detection of an actual mutation. The quality score can be used to distinguish a true positive from a false positive. In some embodiments, in response to the quality score being higher than a predetermined threshold, the processing system can indicate that the individual is statistically likely to have a mutation at the variant location.

### VII. MOMENT MATCHING TO COMBINE STRATIFIED OUTPUTS

FIG. 10 is a flowchart depicting a process for combining outputs of stratified noise models for different read tiers using moment matching, according to some embodiments. The process depicted in FIG. 10 can correspond to steps 950 and/or 960 in FIG. 9. In step 1010, the processing system can combine a mean variant count and a variance of the variant count from each noise model output to produce an overall mean variant count and an overall dispersion parameter. The output of each noise model can take the form of the noise parameters conditioned on stratified sequencing reads. The processing system can evaluate the overall likelihood of the total variant count (e.g., an overall p-value) across different read tiers given the total observed sequencing reads by first matching the individual moments of each read tier to generate overall moments. The processing system can use the overall moments to model an overall distribution conditioned on the total observed sequencing reads. An individual noise distribution for a read tier can be a negative binomial distribution. Likewise, the overall noise distribution across multiple read tiers can also be a negative binomial distribution that matches the moments of the individual read tiers.

The step 1010 can include several sub-steps. For each read tier, the processing system can determine the stratified sequencing depth. The first and second moments of the noise distribution for each tier can be used as the noise parameters to define the noise distribution. In step 1012, based on the stratified sequencing depth, the processing system can determine the first moment (e.g., the mean variant count) of each read tier. For example, in the case of a Bayesian hierarchical model discussed above, the variant frequency for a particular read tier can be modeled as a Gamma-distributed random variable having shape parameter *α_{zₚ,xₚ}* and rate parameter *β_{zₚ,xₚ}* : ${μ}_{p} ∼ Gamma \left({α}_{{z}_{p} , {x}_{p}}, {β}_{{z}_{p} , {x}_{p}}\right)$ Each read tier can have its own shape parameter and rate parameter that are determined based on reference sample datasets. Hence, the variant frequency of each read tier conditioned on the stratified sequencing depth can be different.

The processing system can determine the first moment, the stratified mean variant count, *λₚ*, for each tier by multiplying the variant frequency by the stratified sequencing depth: ${λ}_{p} = {μ}_{p} \times {d}_{{i}_{p}}$

In step 1014, the processing system can also determine the second moment, the variance, of each read tier. In the case of a Bayesian hierarchical model having a Gamma-distributed variant frequency, the variance of each read tier can be determined by the mean variant count, *λₚ*, and a dispersion parameter, *rₚ*. For example, the variance, *vₚ*, can be determined by: ${v}_{p} = {λ}_{p} + \frac{{λ}_{p}^{2}}{{r}_{p}}$

In step 1016, the processing system can determine the overall mean variant count (the overall first moment) and the overall variance (the overall second moment) by moment matching. In some cases, the processing system can perform the moment matching by summing the moments for different read tiers to obtain the overall moment. For example, the overall mean variant count across all read tiers conditioned on the total sequencing depth can be determined by: ${λ}_{all} = \sum {λ}_{p}$ Likewise, the overall variance across all read tiers can be determined by summing the variance of each read tier: ${v}_{all} = \sum {v}_{p}$

The processing system can model the likelihood of the overall observed variant count conditioned on the total sequencing depth by an overall noise distribution. The overall noise distribution can be a negative binomial distribution that is parameterized by the overall mean, *λₐₗₗ*, and an overall dispersion parameter, *rₐₗₗ*. The overall dispersion parameter can be determined by the overall mean and the overall variance: ${r}_{all} = \frac{{λ}_{all}^{2}}{{v}_{all} - {λ}_{all}}$

In step 1020, the processing system can determine the overall likelihood using an overall noise distribution that is modeled by the overall first moment and the overall second moment. For example, the random variable *y_{iₚ}* is modeled as by a negative binomial distribution: $\left.{y}_{{i}_{p}}\right| {d}_{{i}_{p}} ∼ NB \left({λ}_{all}, {r}_{all}\right)$ The random variable *y_{iₚ}*, which represents the likelihood that an event is as or more extreme than the observed overall variant count conditioned on the total sequencing depth, can be the combined result of the processing system. In some cases, the random variable *y_{iₚ}* can be used as the p-value to validate or reject a null hypothesis that the variant reads are due to random events (e.g., noise). The processing system can also apply a negative binomial tail probability to obtain a p-value based on the random variable *y_{iₚ}* and can determine the Phred-scale quality score.

### VIII. INTEGRATION APPROACH TO COMBINE STRATIFIED OUTPUTS

FIG. 11A is a flowchart depicting a process for combining outputs of stratified noise models for different read tiers using an integration approach to combine the likelihoods of each read tiers, according to some embodiments. The processing depicted in FIG. 11A can correspond to steps 950 and/or 960 in FIG. 9. In the process shown in FIG. 11A, the processing system intends to determine a p-value as the combined result of the overall system. The p-value can represent the likelihood that the total variant count for subsequently observed data being greater than or equal to a total variant count observed in the actual data is attributable to noise. Put different, the p-value can represent an event as or more extreme than the observed sequence reads of a subject individual would occur under the null hypothesis. In some cases, an event can be more extreme (i.e., less likely) than the observed variant count of the subject individual when the overall variant count of the event is larger than the observed variant count. Likewise, for a read tier, an event can be more extreme than the stratified observed variant count when the event has a stratified variant count that is higher than the stratified observed variant count. An event with a higher variant count is more extreme because a variant read is often unusual. The chance of observing a variant read at a variant location is often significantly lower than the chance of observing a non-variant read at the variant location.

FIG. 11B illustrates the counting of more extreme events in a multi-dimensional space, in accordance with various embodiments. For simplicity, two dimensions are shown but a processing system in accordance with various embodiments can deal with higher dimensions using the principles illustrated in FIG. 11B. The two dimensions in FIG. 11B can respectively represent two read tiers. For example, the first read tier represents a double-stranded read tier and the second read tier represents a single-stranded read tier.

Focusing on only the read tier for the double-stranded variant count (x-axis) first, in an example, the observed stratified variant count from a subject individual is 2. For the same read tier, an event having a stratified variant count of 3 is less likely (more extreme) than the actual observed stratified variant count because a variant read at a potential variant location is unlikely compared to a non-variant read at the potential variant location. Likely, another event having a stratified variant count of 4 is even less likely than the actual observed stratified variant count. In other words, a combination of the less likely (more extreme) events occupies the space that is larger than the observed variant count and spans all the way to infinity. Conversely, an event having a stratified variant count of 1 or 0 is more likely than the actual observed stratified variant count of 2.

Now considering both read tiers, there can be different combinations of observed stratified variant counts that can be assumed to equally or roughly equally likely. In NGS sample preparation, the nucleic acid sequences of the subject individual can be cleaved in a partially random fashion. As a result, some of the processed sequence reads may not include a complementary sequence read. Hence, some of the processed sequence reads can be single-stranded sequence reads. In other words, for the same nucleic acid sequence sample, different NGS runs will produce different combinations of sequence reads in different read tiers. The stratified variant count of a first read tier can be equivalent to the stratified variant count of a second read tier based on some ratios. In some embodiments, the ratio is modeled as a predetermined value. For example, one double-stranded variant count can be considered to be equivalent to two single-stranded variant count, although in some embodiments numbers other than 2 can also be used.

Based on the observed stratified variant counts of different read tiers, coordinates in the graph shown in FIG. 11B can be divided into points that represent events that are as or more extreme than the observed variant counts of different read tiers and points that represent events that are less extreme than the observed variant counts. For example, assume the actual observed variant count of a subject individual is (1, 2) (i.e., one double-stranded variant count and two single-stranded variant counts). The coordinates (0, 4) and (2, 0) can be assumed to be equivalent to the combination of (1, 2) and represent events that are as extreme as the observed data. All coordinates that are beyond a frontier drawn by the (1, 2), (0, 4), (2, 0) can be classified to be more extreme than the observed data. For example, the coordinate (3, 3) can be considered as a more extreme case. All coordinates that are within the frontier and are closer to the origin can be considered as less extreme (more likely) cases than the observed data. For example, the coordinates (1, 1), (0, 2), (1, 0), etc. can be classified as less extreme than the observed data.

The combined result of the processing system can take the form of the p-value that represents the likelihood of an event that is as or more extreme than the observed data. The processing system can integrate by summing the probabilities corresponding to all coordinates that represent events that are as or more extreme than the observed data to determine the p-value. However, because the coordinates can include points that go all the way to infinity, the processing system can also compute the statistic complement of the p-value instead. In other words, the processing system can sum the probabilities corresponding to all coordinates that represent events that are less extreme than the observed data to determine the complement of the p-value. The processing system can then determine the p-value by subtracting the complement from 1.0. In some embodiments, the processing system can use probabilities in a logarithmic scale for numerical stability because adding floating-point numbers on a computer can be numerically unstable.

Referring back to FIG. 11A, the processing unit can determine the p-value based on the process shown in the figure, according to some embodiments. In step 1110, the processing unit can determine, in each read tier, possible events that are more likely than the observed stratified variant count of the read tier. Those events can define a multidimensional box in which the coordinates can be associated with a greater likelihood than the observed data. The likelihood can be on a logarithmic scale. In step 1120, the processing unit can determine, for one of the plurality of read tiers, whether each of the combinations of stratified variant counts corresponds to a higher or lower likelihood than the observed data. In step 1130, the processing unit can identify combinations of the possible events associated with a higher likelihood of occurrence than the observed stratified variant count of the read tier. The processing unit can repeat steps 1120 and 1130 for each of the read tiers. In step 1140, the processing unit can sum probabilities of the identified combinations to determine a statistic complement. In step 1150, the processing unit can subtract the statistic complement from 1.0 to determine the overall p-value. The p-value can be the combined result that corresponds to step 960 in FIG. 9.

Other ways are also possible for determining the overall p-value. For example, a tail probability technique can be used. In some embodiments, the integration method can be replaced by one or more machine learning models. For example, a random forest regression model can be trained to determine the Phred-scale quality score or the p-value from a set of training sample data. The integration process described in FIG. 11A can be used to generate a plurality of training set samples. The training set samples can be used to train the machine learning model so that the model can be used to determine the quality score.

### IX. EXPERIMENTAL RESULTS

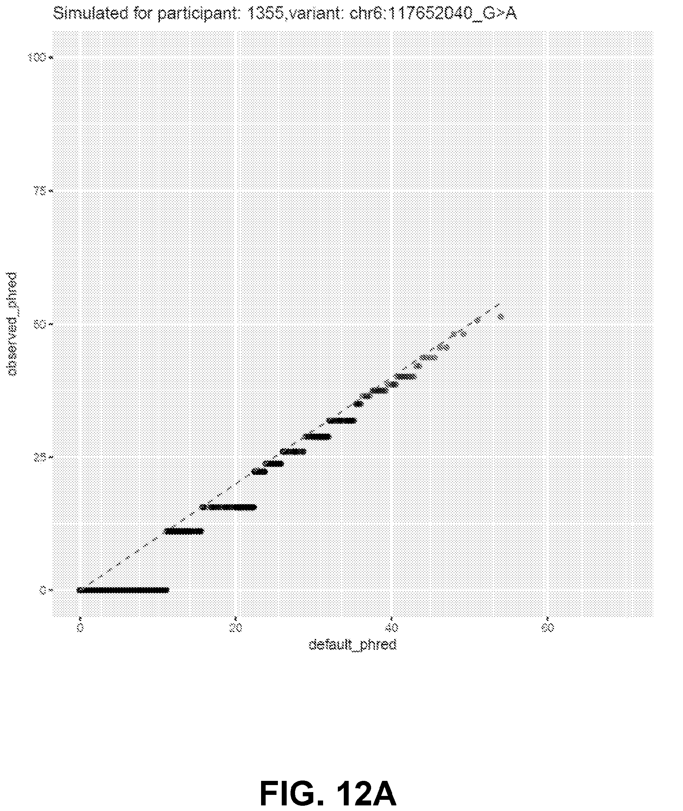

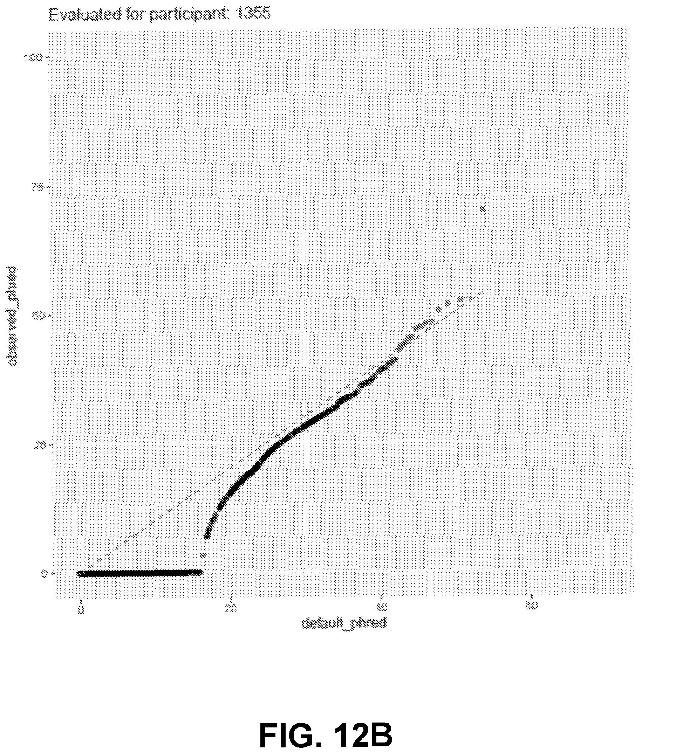

FIGS. 12A and 12B illustrate plots of the observed quality scores against default quality scores in experiments performed using a process according to some embodiments. In FIG. 12A, a calibrated set of simulated data of a sample of a particular individual #1355 is used and analyzed with the tier-level noise model processes described in FIGS. 9 and 11A. The individual has a variant denoted as "chr6" found in the sample. The data simulates random events of variant reads. Each simulated event can have certain variant reads that can be stratified into one or more read tiers. The x-axis represents values of the actual quality score of the simulated event calculated by using the simulated data. The y-axis represents values of the observed quality score determined by the process described in FIG. 11A. The result shows that, except for some discretization, the observed quality scores largely fall on the diagonal line. This indicates that the process described in FIG. 11A successfully determines the likelihood of a possible event that is as or more extreme than the observed data.

In FIG. 12B, the actual dataset of the individual #1355 is used and analyzed with the tier-level noise model processes described in FIGS. 9 and 11A. The dataset can include data of various potential variant locations. Each point corresponds to a potential variant location and the quality score is determined based on the process determined by the process described in FIG. 11A. There are some zero quality score points at the beginning because, in some cases, many of the locations in the actual dataset may have no variant count found in any read tier. Hence, the Phred-scale quality scores equal to zero for all those locations. Most of the rest of the points fall largely along the diagonal line because the individual largely has no mutation at most of the sequence locations. Put differently, the variant counts at those locations that fall along the diagonal line can largely attributable to noise. An outlier having an observed quality score that is significantly higher than the default quality score (e.g. a point at default Phred score of about 55 in FIG. 12B) can indicate that there may be a non-noise event that could be flagged for further evaluation.

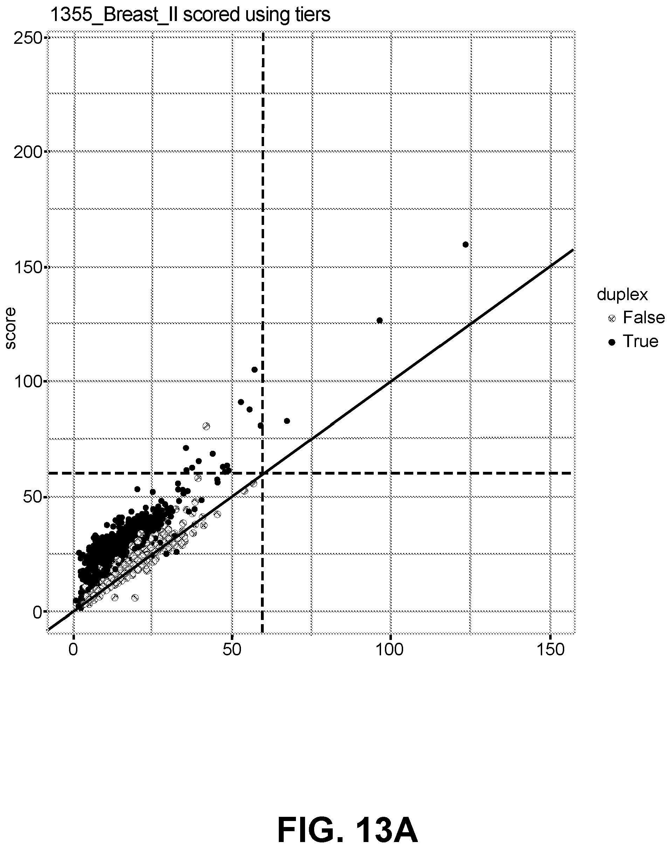

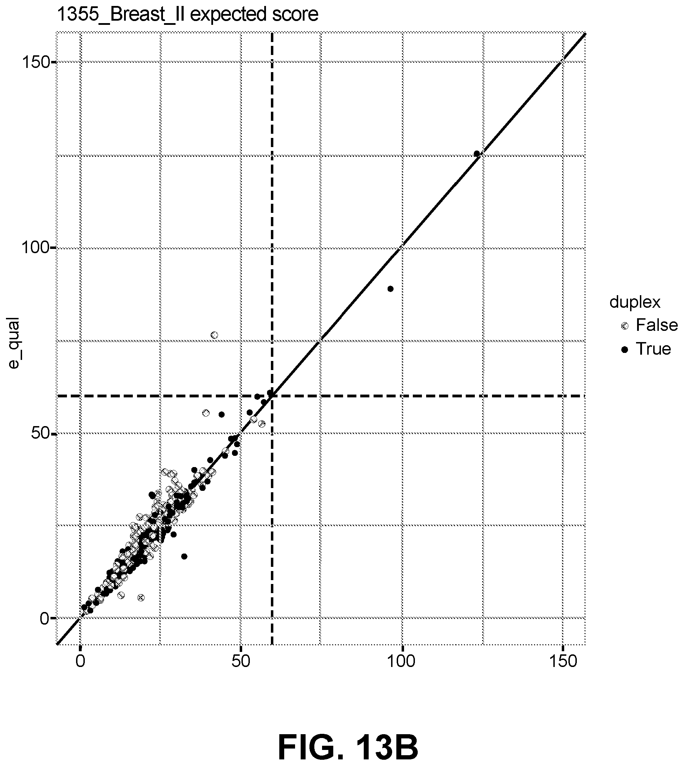

FIGS. 13A and 13B illustrate experimental results that compare the results of quality scores using read tiers to the quality scores using a noise model that does not differentiate the read tiers for any sequence reads or variant reads. The y-axes in FIGS. 13A and 13B represent quality scores determined using methods that stratify sequence reads into different read tiers, in accordance with various embodiments. The x-axes in FIGS. 13A and B represent quality scores determined using a similar noise model, but the noise model does not distinguish sequence reads by read tiers. FIG. 13A illustrates the experimental results of using the integration method described in FIG. 11A to determine the quality scores. The result shows, for sequence reads that include duplex reads (e.g., darker points marked "TRUE"), the data points are shifted upward compared to the sequence reads that include only simplex reads (e.g., lighter points marked "FALSE"). This indicates that the read-tier noise model improves the overall quality scores for sequence reads that include double-stranded reads because double-stranded reads often include more evidence than single-stranded reads. FIG. 13B illustrates the experimental results of using the moment matching method described in FIG. 10 to determine the quality scores. Like FIG. 13A, in this case, the moment matching method also improves the quality scores for sequence reads that include double-stranded reads.

### X. VARIANT IDENTIFICATION

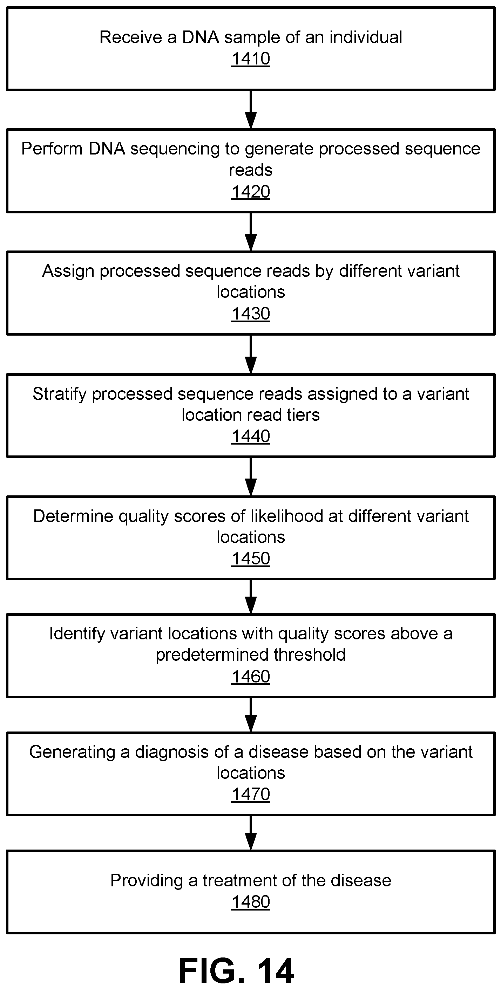

FIG. 14 is a flowchart depicting a process that identifies potential mutation locations of an individual, according to various embodiments. In step 1410, a system can receive a DNA sample of an individual. In step 1420, the system can perform DNA sequencing to generate processed sequence reads. In step 1430, the system can assign the processed sequence reads by different variant locations. In step 1440, for each variant location, the system can stratify the processed sequence reads assigned to the variant location into multiple read tiers. In step 1450, the system can determine the quality score of likelihood at different variant locations. Each quality score can be determined based on the processes described above using noise models that stratify read tiers. In step 1460, the system can identify variant locations with quality scores above a predetermined threshold. Those variant locations can be flagged for further investigation of potential mutation or potential diagnosis.

In step 1470, the system can generate a diagnosis of a disease based on the identified variant locations. In some embodiments, variants or mutations that can be indicative of certain cancers and/or serve as biomarkers for certain therapeutics can include: ACVR1B, AKT3, AMER1, APC, ARID1A, ARID1B, ARID2, ASXL1, ASXL2, ATM, ATR, BAP1 BCL2, BCL6, BCORL1, BCR, BLM, BRAF, BRCA1, BTG1, CASP8, CBL, CCND3, CCNE1, CD74, CDC73, CDK12, CDKN2A, CHD2, CJD2, CREBBP, CSF1R, CTCF, CTNNB1, DICER1, DNAJB1, DNMT1, DNMT3A, DNMT3B, DOT1L, EED, EGFR, EIF1AX, EP300, EPHA3, EPHA5, EPHB1, ERBB2, ERBB4, ERCC2, ERCC3, ERCC4, ESR1, FAM46C, FANCA, FANCC, FANCD2, FANCE, FAT1, FBXW7, FGFR3, FLCN, FLT1, FOXO1, FUBP1, FYN, GATA3, GPR124, GRIN2A, GRM3, H3F3A, HIST1H1C,IDH1, IDH2, IKZF1, IL7R, INPP4B, IRF4, IRS1, IRS2, JAK2, KAT6A, KDM6A, KEAP1, KIF5B, KIT, KLF4, KLH6, KMT2C, KRAS, LMAP1, LRP1B, LZTR1, MAP3K1, MCL1, MGA, MSH2, MSH6, MST1R, MTOR, MYD88, NPM1, NRAS, NTRK1, NTRK2, NUP93, NUTM1, PAX3, PAX8, PBRM1, PGR, PHOX2B, PIK3CA, POLE, PTCH1, PTEN, PTPN11, PTPRT, RAD21, RAF1, RANBP2, RB1, REL, RFWD2, RHOA, RPTOR, RUNX1, RUNX1T1, SDHA, SHQ1, SLIT2, SMAD4, SMARCA4, SMARCD1, SNCAIP, SOCS1, SPEN, SPTA1, SUZ12, TET1, TET2, TGFBR, and TNFRSF14. In some embodiments, cancer immunotherapies can target OX40, LAG3, and/or ICOS.

In step 1480, a treatment of the disease may be provided. Before providing the treatment, a companion diagnostics operation may also be performed. The companion diagnostics operation may identify one or more criteria, including variants or mutations, using the process described herein. Providing the treatment may take the form of causing or recommending a medical practitioner to administrate a specific dosage of a drug to a patient.

For example, the systems and methods described herein can be used for detecting variants or mutations that are biomarkers for cancer treatments, such as certain immunotherapies and targeted therapeutics. Such therapies can include, for example, an immunoglobulin, a protein, a peptide, a small molecule, a nanoparticle, or a nucleic acid. In some embodiments, the therapies comprise an antibody, or a functional fragment thereof. In some embodiments, the antibody may include: Rituxan^{®} (rituximab), Herceptin^{®} (trastuzumab), Erbitux^{®} (cetuximab), Vectibix^{®} (Panitumumab), Arzerra^{®} (Ofatumumab), Benlysta^{®} (belimumab), Yervoy^{®} (ipilimumab), Perjeta^{®} (Pertuzumab), Tremelimumab^{®}, Opdivo^{®} (nivolumab), Dacetuzumab^{®}, Urelumab^{®}, Tecentriq^{®} (atezolizumab, MPDL3280A), Lambrolizumab^{®}, Blinatumomab^{®}, CT-011, Keytruda^{®} (pembrolizumab, MK-3475), BMS-936559, MED14736, MSB0010718C, Imfinzi^{®} (durvalumab), Bavencio^{®} (avelumab) and margetuximab (MGAH22).

In some embodiments, the immunotherapies and targeted therapeutics comprise PD-1 inhibition, PD-L1 inhibition, or CTL-4 inhibition. PD-1 inhibition targets the programmed death receptor on T-cells and other immune cells. Examples of PD-1 inhibition immunotherapies include Pembrolizumab; Keytruda; Nivolumab; Opdivo; Cemiplimab; Libtayo. PD-L1 inhibition targets the programmed death receptor ligand expressed by tumor and regulatory immune cells. Examples of PD-L1 Inhibition immunotherapies include Atezolizumab; Tecentriq; Avelumab; Bavencio; Durvalumab; Imfinzi. CTL-4 inhibition targets T-cell activation. Examples of CTL-4 inhibition immunotherapies include Ipilimumab; Yervoy.

For non-small cell lung cancer indications, variants or mutations that can be biomarkers for immunotherapy treatments can include EGFR exon 19 deletions & EGFR exon 21 L858R alterations (e.g., for therapies such as Gilotrif^{®} (afatinib), Iressa^{®} (gefitinib), Tagrisso^{®} (osimertinib), or Tarceva^{®} (erlotinib)); EGFR exon 20 T790M alterations (e.g., which may be treated with Tagrisso^{®} (osimertinib)); ALK rearrangements (e.g., which may be treated with Alecensa^{®} (alectinib), Xalkori^{®} (crizotinib), or Zykadia^{®} (ceritinib)); BRAF V600E (e.g., which may be treated with Tafinlar^{®} (dabrafenib) in combination with Mekinist^{®} (trametinib)); single nucleotide variants (SNVs) and indels that lead to MET exon 14 skipping (e.g., which may be treated with TabrectaTM (capmatinib)).

For melanoma indications, variants or mutations that can be biomarkers for immunotherapy treatments can include BRAF V600E (e.g., which may be treated with Tafinlar^{®} (dabrafenib) or Zelboraf^{®} (vemurafenib)); BRAF V600E or V600K (e.g., which may be treated with Mekinist^{®} (trametinib) or Cotellic^{®} (cobimetinib), in combination with Zelboraf^{®} (vemurafenib)).

For breast cancer indications, variants or mutations that can be biomarkers for immunotherapy treatments can include ERBB2 (HER2) amplification (e.g., which may be treated with Herceptin^{®} (trastuzumab), Kadcyla^{®} (ado-trastuzumab-emtansine), or Perjeta^{®} (pertuzumab)); PIK3CA alterations (e.g., which may be treated with Piqray^{®} (alpelisib)).

For colorectal cancer indications, variants or mutations that can be biomarkers for immunotherapy treatments can include KRAS wild-type (absence of mutations in codons 12 and 13) (e.g., which may be treated with Erbitux^{®} (cetuximab)); KRAS wild-type (absence of mutations in exons 2, 3, and 4) and NRAS wild type (absence of mutations in exons 2, 3, and 4) (e.g., which may be treated with Vectibix^{®} (panitumumab)).

For ovarian cancer indications, variants or mutations that can be biomarkers for immunotherapy treatments can include BRCA1/2 alterations (e.g., which may be treated with Lynparza^{®} (olaparib) or Rubraca^{®} (rucaparib)).

For prostate cancer indications, variants or mutations that can be biomarkers for immunotherapy treatments can include Homologous Recombination Repair (HRR) gene (BRCA1, BRCA2, ATM, BARD1, BRIP1, CDK12, CHEK1, CHEK2, FANCL, PALB2, RAD51B, RAD51C, RAD51D and RAD54L) alterations (e.g., which may be treated with Lynparza^{®} (olaparib)).

For solid tumor cancer indications, variants or mutations that can be biomarkers for immunotherapy treatments can include a tumor mutational burden (TMB) that is greater than or equal to 10 mutations per megabase (e.g., which may be treated with Keytruda^{®} (pembrolizumab)).

### XI. COMPUTING MACHINE ARCHITECTURE

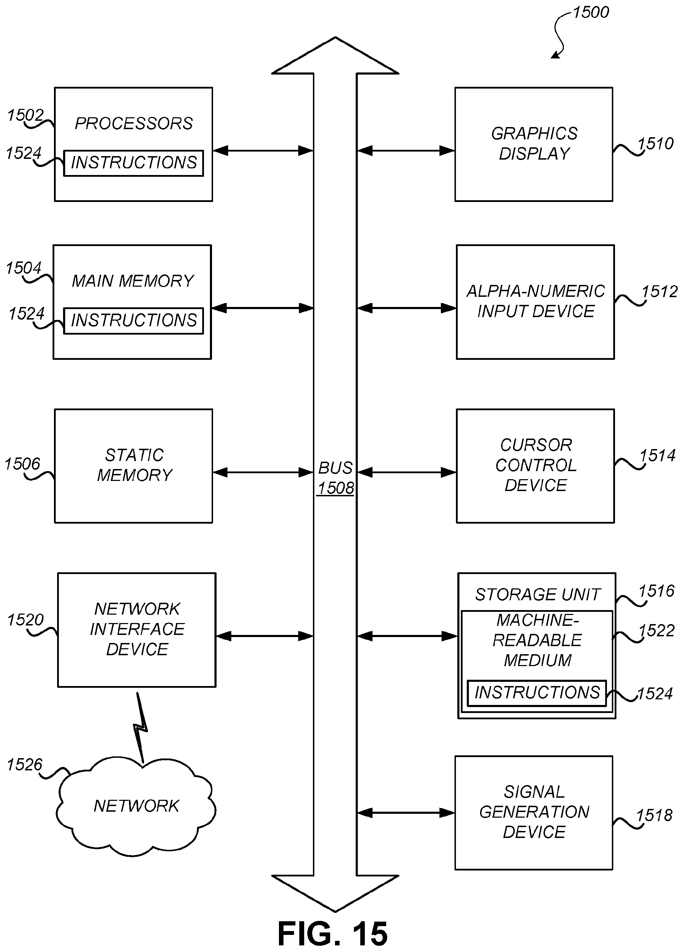

FIG. 15 is a block diagram illustrating components of an example computing machine that is capable of reading instructions from a computer-readable medium and execute them in a processor (or controller). A computer described herein may include a single computing machine shown in FIG. 15, a virtual machine, a distributed computing system that includes multiples nodes of computing machines shown in FIG. 15, or any other suitable arrangement of computing devices.

By way of example, FIG. 15 shows a diagrammatic representation of a computing machine in the example form of a computer system 1500 within which instructions 1524 (*e.g*., software, program code, or machine code), which may be stored in a computer-readable medium for causing the machine to perform any one or more of the processes discussed herein may be executed. In some embodiments, the computing machine operates as a standalone device or may be connected (*e.g*., networked) to other machines. In a networked deployment, the machine may operate in the capacity of a server machine or a client machine in a server-client network environment, or as a peer machine in a peer-to-peer (or distributed) network environment.

The structure of a computing machine described in FIG. 15 may correspond to any software, hardware, or combined components (e.g. those shown in FIG. 2 or a processing unit described herein), including but not limited to any engines, modules, computing server, machines that are used to perform one or more processes described herein. While FIG. 15 shows various hardware and software elements, each of the components described herein may include additional or fewer elements.

By way of example, a computing machine may be a personal computer (PC), a tablet PC, a set-top box (STB), a personal digital assistant (PDA), a cellular telephone, a smartphone, a web appliance, a network router, an internet of things (IoT) device, a switch or bridge, or any machine capable of executing instructions 1524 that specify actions to be taken by that machine. Further, while only a single machine is illustrated, the term "machine" and "computer" may also be taken to include any collection of machines that individually or jointly execute instructions 1524 to perform any one or more of the methodologies discussed herein.

The example computer system 1500 includes one or more processors 1502 such as a CPU (central processing unit), a GPU (graphics processing unit), a TPU (tensor processing unit), a DSP (digital signal processor), a system on a chip (SOC), a controller, a state equipment, an application-specific integrated circuit (ASIC), a field-programmable gate array (FPGA), or any combination of these. Parts of the computing system 1500 may also include a memory 1504 that store computer code including instructions 1524 that may cause the processors 1502 to perform certain actions when the instructions are executed, directly or indirectly by the processors 1502. Instructions can be any directions, commands, or orders that may be stored in different forms, such as equipment-readable instructions, programming instructions including source code, and other communication signals and orders. Instructions may be used in a general sense and are not limited to machine-readable codes.

One and more methods described herein improve the operation speed of the processors 1502 and reduces the space required for the memory 1504. For example, the machine learning methods described herein reduces the complexity of the computation of the processors 1502 by applying one or more novel techniques that simplify the steps in training, reaching convergence, and generating results of the processors 1502. The algorithms described herein also reduces the size of the models and datasets to reduce the storage space requirement for memory 1504.

The performance of certain of the operations may be distributed among the more than processors, not only residing within a single machine, but deployed across a number of machines. In some example embodiments, the one or more processors or processor-implemented modules may be located in a single geographic location (*e.g*., within a home environment, an office environment, or a server farm). In other example embodiments, the one or more processors or processor-implemented modules may be distributed across a number of geographic locations. Even though in the specification or the claims may refer some processes to be performed by a processor, this should be construed to include a joint operation of multiple distributed processors.

The computer system 1500 may include a main memory 1504, and a static memory 1506, which are configured to communicate with each other via a bus 1508. The computer system 1500 may further include a graphics display unit 1510 (*e.g.,* a plasma display panel (PDP), a liquid crystal display (LCD), a projector, or a cathode ray tube (CRT)). The graphics display unit 1510, controlled by the processors 1502, displays a graphical user interface (GUI) to display one or more results and data generated by the processes described herein. The computer system 1500 may also include alphanumeric input device 1512 (*e.g.,* a keyboard), a cursor control device 1514 (*e.g.,* a mouse, a trackball, a joystick, a motion sensor, or other pointing instrument), a storage unit 1516 (a hard drive, a solid state drive, a hybrid drive, a memory disk, etc.), a signal generation device 1518 (*e.g.,* a speaker), and a network interface device 1520, which also are configured to communicate via the bus 1508.

The storage unit 1516 includes a computer-readable medium 1522 on which is stored instructions 1524 embodying any one or more of the methodologies or functions described herein. The instructions 1524 may also reside, completely or at least partially, within the main memory 1504 or within the processor 1502 (*e.g*., within a processor's cache memory) during execution thereof by the computer system 1500, the main memory 1504 and the processor 1502 also constituting computer-readable media. The instructions 1524 may be transmitted or received over a network 1526 via the network interface device 1520. While computer-readable medium 1522 is shown in an example embodiment to be a single medium, the term "computer-readable medium" should be taken to include a single medium or multiple media (*e.g.,* a centralized or distributed database, or associated caches and servers) able to store instructions (*e.g*., instructions 1524). The computer-readable medium may include any medium that is capable of storing instructions (*e.g*., instructions 1524) for execution by the processors (e.g., processors 1502) and that cause the processors to perform any one or more of the methodologies disclosed herein. The computer-readable medium may include, but not be limited to, data repositories in the form of solid-state memories, optical media, and magnetic media. The computer-readable medium does not include a transitory medium such as a propagating signal or a carrier wave.

### XII. ADDITIONAL CONSIDERATIONS

Beneficially, various embodiments described herein improve the accuracy and efficiency of existing technologies in the field of sequencing, such as PCR and massively parallel DNA sequencing (e.g., NGS). The embodiments provide solutions to the challenge of identifying errors introduced by the sequencing and amplification process. A massively parallel DNA sequencing may start with one or more DNA samples, which are randomly cleaved and typically amplified using PCR. The parallel nature of massively parallel DNA sequencing results in replicates of nucleotide sequences of each allele. The extent of replication and sequencing at each allele site could vary. For example, some sequences are overlapped and/or double-stranded while other sequences are not. Both the PCR amplification process and the sequencing process and the sequencing process have non-trivial error rates. The sequence errors may act to obscure the nucleotide sequences of the true alleles. The embodiments may be used to determine one or more alleles analyzed by a massively parallel DNA sequencing instrument. By taking consideration of read-tier specific noise models, the massively parallel DNA sequencing workflows exhibits sufficient fidelity to generate correct sequence determination by more accurately distinguishing true alleles from erroneous sequences.

Conventionally, to reduce the error rate in determining the correct sequence, the sequencing depth of the sample needs to increase. This means fewer samples can be analyzed in a batch of sequencing because more resources are dedicated to a sample. The embodiments improve the accuracy of sequencing without increasing the sequencing depth of a particular allele site, thereby allowing more allele sites or patient samples to be sequenced at the same time in the case of a massively parallel DNA sequencing. Embodiments described may reduce the sequencing depths needed while increasing the accuracy of a massively parallel DNA sequencing that is used to read the nucleotide sequences generated in the amplification.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above disclosure.

Some portions of this description describe the embodiments of the invention in terms of algorithms and symbolic representations of operations on information. These algorithmic descriptions and representations are commonly used by those skilled in the data processing arts to convey the substance of their work effectively to others skilled in the art. These operations, while described functionally, computationally, or logically, are understood to be implemented by computer programs or equivalent electrical circuits, microcode, or the like. Furthermore, it has also proven convenient at times, to refer to these arrangements of operations as modules, without loss of generality. The described operations and their associated modules may be embodied in software, firmware, hardware, or any combinations thereof.

Any of the steps, operations, or processes described herein may be performed or implemented with one or more hardware or software modules, alone or in combination with other devices. In some embodiments, a software module is implemented with a computer program product including a non-transitory computer-readable medium containing computer program code, which can be executed by a computer processor for performing any or all of the steps, operations, or processes described.

Embodiments of the invention may also relate to a product that is produced by a computing process described herein. Such a product may include information resulting from a computing process, where the information is stored on a non-transitory, tangible computer readable storage medium and may include any embodiments of a computer program product or other data combination described herein.

While one or more processes described herein may be described with one or more steps, the use of the term "step" does not imply that a particular order. For example, while this disclosure may describe a process that includes multiple steps sequentially, the steps in the process do not need to be performed by the specific order claimed or described in the disclosure. Some steps may be performed before others even though the other steps are claimed or described first in this disclosure.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based hereon. Accordingly, the disclosure of the embodiments of the invention is intended to be illustrative, but not limiting, of the scope of the invention, which is set forth in the following claims.

## Claims

1. A computer-implemented method for processing a DNA sequencing dataset of a sample, the computer-implemented method comprising:
accessing the DNA sequencing dataset generated by a DNA sequencing, the DNA sequencing dataset comprising a plurality of processed sequence reads that include a variant location;
stratifying the plurality of processed sequence reads into a plurality of read tiers, wherein the plurality of read tiers include one or more of:
a) a double-stranded, stitched read tier,
b) a double-stranded, unstitched read tier,
c) a single-stranded, stitched read tier, and
d) a single-stranded, unstitched read tier;
determining, for each read tier, a stratified sequencing depth at the variant location;
determining, for each read tier, one or more noise parameters conditioned on the stratified sequencing depth of the read tier, the one or more noise parameters corresponding to a noise model specific to the read tier, wherein training the noise model comprises:
stratifying training DNA datasets of a plurality of reference healthy individuals,
selecting stratified sequence reads for the read tier as a stratified training set,
initiating the one or more noise parameters that model a noise distribution that represents the noise model, and
iteratively adjusting values of the one or more noise parameters based on the noise distribution of the stratified training set from the plurality of reference healthy individuals;
generating, for each read tier, an output of the noise model specific to the read tier based on the one or more noise parameters conditioned on the stratified sequencing depth of the read tier; and
combining the generated noise model outputs to produce a combined result representative of a likelihood that the sample is associated with a total variant count, wherein combining the generated noise model outputs to produce the combined result comprises:
determining an observed stratified variant count of each read tier;
determining in each read tier, possible events that are more likely than the observed stratified variant count of each read tier; and
identifying combinations of the possible events associated with a higher likelihood of occurrence than the observed stratified variant count of each read tier.

2. The computer-implemented method of claim 1, wherein a mutation at the variant location is one of: a single nucleotide variant, an insertion, and a deletion.

3. The computer-implemented method of claims 1 or 2, further comprising:
determining a quality score of the combined result, the quality score being a Phred-scale score.

4. The computer-implemented method of any of claims 1 to 3, wherein determining, for a read tier, the one or more noise parameters conditioned on the stratified sequencing depth of the read tier comprises:
accessing a parameter distribution specific to the read tier, the parameter distribution describing a distribution of a set of DNA sequencing samples associated with the read tier, wherein the noise parameters are determined from the parameter distribution.

5. The computer-implemented method of any of claims 1 to 4, wherein a first noise parameter corresponding to a noise model specific to a first read tier has a different value than a corresponding second noise parameter corresponding to a noise model specific to a second read tier.

6. The computer-implemented method of any of claims 1 to 5, wherein, for each read tier, the determined one or more noise parameters comprise a mean of the noise distribution conditioned on the stratified sequencing depth of the read tier.

7. The computer-implemented method of any of claims 1 to 6, wherein the generated output of each noise model is the one or more noise parameters conditioned on the stratified sequencing depth determined for the read tier.

8. The computer-implemented method of any of claims 1 to 7, wherein the generated output of each noise model comprises a likelihood that a stratified variant count for the read tier exceeds a threshold.

9. The computer-implemented method of any of claims 1 to 8, wherein combining the generated noise model outputs comprises combining a mean variant count and a variance from each noise model output to produce an overall mean variant count and the overall dispersion parameter representative of an overall noise distribution for the combined result.

10. The computer-implemented method of any of claims 1 to 9, wherein combining the generated noise model outputs to produce the combined result further comprises:
summing probabilities of the identified combinations to determine a statistic complement; and
determining a likelihood value by subtracting the statistic complement from 1.0.

11. The computer-implemented method of any of claims 1 to 10, further comprising:
receiving a body fluid sample of an individual;
performing the DNA sequencing on cfDNA of the body fluid sample;
generating raw sequence reads based on a result of the DNA sequencing; and
collapsing and stitching the raw sequence reads to generate the plurality of processed sequence reads.

12. The computer-implemented method of any of claims 1 to 11, wherein the DNA sequencing comprises a massively parallel DNA sequencing operation.

13. The computer-implemented method of any of claims 1 to 12, further comprising:
providing, based on the combined result, a diagnosis of a subject having a variant.

14. The computer-implemented method of claim 13, wherein the variant is selected from the group consisting of: ACVR1B, AKT3, AMER1, APC, ARID1A, ARID1B, ARID2, ASXL1, ASXL2, ATM, ATR, BAP1 BCL2, BCL6, BCORL1, BCR, BLM, BRAF, BRCA1, BTG1, CASP8, CBL, CCND3, CCNE1, CD74, CDC73, CDK12, CDKN2A, CHD2, CJD2, CREBBP, CSF1R, CTCF, CTNNB1, DICER1, DNAJB1, DNMT1, DNMT3A, DNMT3B, DOT1L, EED, EGFR, EIF1AX, EP300, EPHA3, EPHA5, EPHB1, ERBB2, ERBB4, ERCC2, ERCC3, ERCC4, ESR1, FAM46C, FANCA, FANCC, FANCD2, FANCE, FAT1, FBXW7, FGFR3, FLCN, FLT1, FOXO1, FUBP1, FYN, GATA3, GPR124, GRIN2A, GRM3, H3F3A, HIST1H1C,IDH1, IDH2, IKZF1, IL7R, INPP4B, IRF4, IRS1, IRS2, JAK2, KAT6A, KDM6A, KEAP1, KIF5B, KIT, KLF4, KLH6, KMT2C, KRAS, LMAP1, LRP1B, LZTR1, MAP3K1, MCL1, MGA, MSH2, MSH6, MST1R, MTOR, MYD88, NPM1, NRAS, NTRK1, NTRK2, NUP93, NUTM1, PAX3, PAX8, PBRM1, PGR, PHOX2B, PIK3CA, POLE, PTCH1, PTEN, PTPN11, PTPRT, RAD21, RAF1, RANBP2, RB1, REL, RFWD2, RHOA, RPTOR, RUNX1, RUNX1T1, SDHA, SHQ1, SLIT2, SMAD4, SMARCA4, SMARCD1, SNCAIP, SOCS1, SPEN, SPTA1, SUZ12, TET1, TET2, TGFBR, and TNFRSF14.

## Patentansprüche

1. Computerimplementiertes Verfahren zum Verarbeiten eines DNA-Sequenzierungsdatensatzes einer Probe, wobei das computerimplementierte Verfahren Folgendes umfasst:
Zugreifen auf den durch eine DNA-Sequenzierung erzeugten DNA-Sequenzierungsdatensatz, wobei der DNA-Sequenzierungsdatensatz eine Vielzahl von verarbeiteten Sequenzlesungen umfasst, die eine Variantenstelle beinhalten;
Stratifizieren der Vielzahl von verarbeiteten Sequenzlesungen in eine Vielzahl von Leseebenen, wobei die Vielzahl von Leseebenen eines oder mehrere der Folgenden beinhalten:
a) eine doppelsträngige, zusammengefügte Leseebene,
b) eine doppelsträngige, nicht-zusammengefügte Leseebene,
c) eine einzelsträngige, zusammengefügte Leseebene, und
d) eine einzelsträngige, nicht-zusammengefügte Leseebene;
Bestimmen, für jede Leseebene, einer stratifizierten Sequenziertiefe an der Variantenstelle;
Bestimmen, für jede Leseebene, eines oder mehrerer Rauschparameter, die auf die stratifizierte Sequenziertiefe der Leseebene konditioniert sind, wobei der eine oder die mehreren Rauschparameter einem Rauschmodell entsprechen, das für die Leseebene spezifisch ist, wobei das Trainieren des Rauschmodells Folgendes umfasst:
Stratifizieren von Trainings-DNA-Datensätzen einer Vielzahl gesunder Referenzindividuen,
Auswählen von stratifizierten Sequenzlesungen für die Leseebene als stratifizierter Trainingssatz,
Initiieren des einen oder der mehreren Rauschparameter, die eine Rauschverteilung modellieren, die das Rauschmodell repräsentiert, und
Iteratives Anpassen von Werten des einen oder der mehreren Rauschparameter basierend auf der Rauschverteilung des stratifizierten Trainingssatzes aus der Vielzahl von gesunden Referenzindividuen;
Erzeugen, für jede Leseebene, einer Ausgabe des Rauschmodells, das für die Leseebene spezifisch ist, basierend auf dem einen oder den mehreren Rauschparametern, die auf die stratifizierte Sequenziertiefe der Leseebene konditioniert sind; und
Kombinieren der erzeugten Rauschmodellausgaben zum Erstellen eines kombinierten Ergebnisses, das eine Wahrscheinlichkeit repräsentiert, dass die Probe mit einer Gesamtvariantenzählung assoziiert ist, wobei das Kombinieren der erzeugten Rauschmodellausgaben zum Erstellen des kombinierten Ergebnisses Folgendes umfasst:
Bestimmen einer beobachteten stratifizierten Variantenzählung jeder Leseebene;
in jeder Leseebene Bestimmen von möglichen Ereignisse, die wahrscheinlicher als die beobachtete stratifizierte Variantenzählung jeder Leseebene sind; und
Identifizieren von Kombinationen der möglichen Ereignisse, die mit einer höheren Wahrscheinlichkeit des Auftretens assoziiert sind als die beobachtete stratifizierte Variantenzählung jeder Leseebene.

2. Computerimplementiertes Verfahren nach Anspruch 1, wobei eine Mutation an der Variantenstelle eine der Folgenden ist: eine einzelne Nukleotidvariante, eine Insertion und eine Deletion.

3. Computerimplementiertes Verfahren nach Anspruch 1 oder 2, das ferner umfasst:
Bestimmen einer Qualitätsbewertung des kombinierten Ergebnisses, wobei die Qualitätsbewertung ein Phred-Scale-Score ist.

4. Computerimplementiertes Verfahren nach einem der Ansprüche 1 bis 3, wobei das Bestimmen, für eine Leseebene, des einen oder der mehreren Rauschparameter, die auf die stratifizierte Sequenziertiefe der Leseebene konditioniert sind, Folgendes umfasst:
Zugreifen auf eine für die Leseebene spezifisch Parameterverteilung, wobei die Parameterverteilung eine Verteilung eines Satzes von DNA-Sequenzierproben, assoziiert mit der Leseebene, beschreibt, wobei die Rauschparameter aus der Parameterverteilung bestimmt werden.

5. Computerimplementiertes Verfahren nach einem der Ansprüche 1 bis 4, wobei ein erster Rauschparameter, der einem für eine erste Leseebene spezifischen Rauschmodell entspricht, einen anderen Wert aufweist als ein entsprechender zweiter Rauschparameter, der einem für eine zweite Leseebene spezifischen Rauschmodell entspricht.

6. Computerimplementiertes Verfahren nach einem der Ansprüche 1 bis 5, wobei für jede Leseebene der eine bestimmte oder die mehreren bestimmten Rauschparameter einen Mittelwert der Rauschverteilung, konditioniert auf die stratifizierte Sequenziertiefe der Leseebene, umfassen.

7. Computerimplementiertes Verfahren nach einem der Ansprüche 1 bis 6, wobei es sich bei der erzeugten Ausgabe jedes Rauschmodells um den einen oder die mehreren Rauschparameter handelt, konditioniert auf die stratifizierte Sequenziertiefe, die für die Leseebene bestimmt wird.

8. Computerimplementiertes Verfahren nach einem der Ansprüche 1 bis 7, wobei die erzeugte Ausgabe jedes Rauschmodells eine Wahrscheinlichkeit umfasst, dass eine stratifizierte Variantenzählung für die Leseebene eine Schwelle überschreitet.

9. Computerimplementiertes Verfahren nach einem der Ansprüche 1 bis 8, wobei das Kombinieren der erzeugten Rauschmodellausgaben das Kombinieren einer mittleren Variantenzählung und einer Varianz aus jeder Rauschmodellausgabe umfasst, um eine gesamte mittlere Variantenzählung und den Gesamtdispersionsparameter, repräsentativ für eine Gesamtrauschverteilung für das kombinierte Ergebnis, zu erstellen.

10. Computerimplementiertes Verfahren nach einem der Ansprüche 1 bis 9, wobei das Kombinieren der erzeugten Rauschmodellausgaben zum Erstellen des kombinierten Ergebnisses ferner umfasst:
Summieren von Wahrscheinlichkeiten der identifizierten Kombinationen, um ein statistisches Komplement zu bestimmen; und
Bestimmen eines Wahrscheinlichkeitswerts durch Subtrahieren des statistischen Komplements von 1,0.

11. Computerimplementiertes Verfahren nach einem der Ansprüche 1 bis 10, ferner umfassend:
Empfangen einer Körperflüssigkeitsprobe eines Individuums;
Durchführen der DNA-Sequenzierung an cfDNA der Körperflüssigkeitsprobe;
Erzeugen von Rohsequenzlesungen basierend auf einem Ergebnis der DNA-Sequenzierung; und
Zusammenlegen und Zusammenfügen der rohen Sequenzlesungen, um die Vielzahl von verarbeiteten Sequenzlesungen zu erzeugen.

12. Computerimplementiertes Verfahren nach einem der Ansprüche 1 bis 11, wobei die DNA-Sequenzierung eine massiv parallele DNA-Sequenzierungsoperation umfasst.

13. Computerimplementiertes Verfahren nach einem der Ansprüche 1 bis 12, ferner umfassend:
Bereitstellen, basierend auf dem kombinierten Ergebnis, einer Diagnose für ein Individuum, das eine Variante aufweist.

14. Computerimplementiertes Verfahren nach Anspruch 13, wobei die Variante ausgewählt ist aus der Gruppe bestehend aus: ACVR1B, AKT3, AMER1, APC, ARID1A, ARID1B, ARID2, ASXL1, ASXL2, ATM, ATR, BAP1 BCL2, BCL6, BCORL1, BCR, BLM, BRAF, BRCA1, BTG1, CASP8, CBL, CCND3, CCNE1, CD74, CDC73, CDK12, CDKN2A, CHD2, CJD2, CREBBP, CSF1R, CTCF, CTNNB1, DICER1, DNAJB1, DNMT1, DNMT3A, DNMT3B, DOT1L, EED, EGFR, EIF1AX, EP300, EPHA3, EPHA5, EPHB1, ERBB2, ERBB4, ERCC2, ERCC3, ERCC4, ESR1, FAM46C, FANCA, FANCC, FANCD2, FANCE, FAT1, FBXW7, FGFR3, FLCN, FLT1, FOXO1, FUBP1, FYN, GATA3, GPR124, GRIN2A, GRM3, H3F3A, HIST1H1C, IDH1, IDH2, IKZF1, IL7R, INPP4B, IRF4, IRS1, IRS2, JAK2, KAT6A, KDM6A, KEAP1, KIF5B, KIT, KLF4, KLH6, KMT2C, KRAS, LMAP1, LRP1B, LZTR1, MAP3K1, MCL1, MGA, MSH2, MSH6, MST1R, MTOR, MYD88, NPM1, NRAS, NTRK1, NTRK2, NUP93, NUTM1, PAX3, PAX8, PBRM1, PGR, PHOX2B, PIK3CA, POLE, PTCH1, PTEN, PTPN11, PTPRT, RAD21, RAF1, RANBP2, RB1, REL, RFWD2, RHOA, RPTOR, RUNX1, RUNX1T1, SDHA, SHQ1, SLIT2, SMAD4, SMARCA4, SMARCD1, SNCAIP, SOCS1, SPEN, SPTA1, SUZ12, TET1, TET2, TGFBR et TNFRSF14.

## Revendications

1. Procédé mis en œuvre par ordinateur pour traiter un ensemble de données de séquençage d'ADN d'un échantillon, le procédé mis en œuvre par ordinateur comprenant :
l'accès à l'ensemble de données de séquençage d'ADN généré par un séquençage d'ADN, l'ensemble de données de séquençage d'ADN comprenant une pluralité de lectures de séquences traitées qui comprennent une position de variant ;
la stratification de la pluralité de lectures de séquences traitées en une pluralité de niveaux de lecture, la pluralité de niveaux de lecture comprenant un ou plusieurs des éléments suivants :
a) un niveau de lecture double brin, fusionné,
b) un niveau de lecture double brin, non fusionné,
c) un niveau de lecture simple brin, fusionné, et
d) un niveau de lecture simple brin, non fusionné ;
la détermination, pour chaque niveau de lecture, d'une profondeur de séquençage stratifiée à la position de variant ;
la détermination, pour chaque niveau de lecture, d'un ou plusieurs paramètres de bruit conditionnés par la profondeur de séquençage stratifiée du niveau de lecture, le ou les paramètres de bruit correspondant à un modèle de bruit spécifique au niveau de lecture, dans lequel l'entraînement du modèle de bruit comprend :
la stratification des ensembles de données d'ADN d'entraînement d'une pluralité d'individus sains de référence,
la sélection des lectures de séquence stratifiées pour le niveau de lecture en tant qu'ensemble d'entraînement stratifié,
l'initialisation du ou des paramètres de bruit qui modélisent une distribution de bruit qui représente le modèle de bruit, et
l'ajustement itératif des valeurs du ou des paramètres de bruit sur la base de la distribution de bruit de l'ensemble d'entraînement stratifié de la pluralité d'individus sains de référence ;
la génération, pour chaque niveau de lecture, d'une sortie du modèle de bruit spécifique au niveau de lecture sur la base du ou des paramètres de bruit conditionnés par la profondeur de séquençage stratifiée du niveau de lecture ; et
la combinaison des sorties de modèle de bruit générées pour produire un résultat combiné représentatif d'une probabilité que l'échantillon soit associé à un nombre total de variants, la combinaison des sorties du modèle de bruit généré pour produire le résultat combiné comprenant :
la détermination d'un nombre de variants stratifiés observés de chaque niveau de lecture ;
la détermination, dans chaque niveau de lecture, des événements possibles qui sont plus probables que le nombre de variants stratifiés observés de chaque niveau de lecture ; et
l'identification de combinaisons des événements possibles associés à une probabilité d'occurrence plus élevée que le nombre de variants stratifiés observés de chaque niveau de lecture.

2. Procédé mis en œuvre par ordinateur selon la revendication 1, dans lequel une mutation à la position de variant est soit : un variant nucléotidique unique, une insertion ou une délétion.

3. Procédé mis en œuvre par ordinateur selon la revendication 1 ou 2, comprenant en outre :
la détermination d'un score de qualité du résultat combiné, le score de qualité étant un score d'échelle Phred.

4. Procédé mis en œuvre par ordinateur selon l'une quelconque des revendications 1 à 3, dans lequel la détermination, pour un niveau de lecture, du ou des paramètres de bruit conditionnés par la profondeur de séquençage stratifiée du niveau de lecture, comprend :
l'accès à une distribution de paramètres spécifique au niveau de lecture, la distribution de paramètres décrivant une distribution d'un ensemble d'échantillons de séquençage d'ADN associés au niveau de lecture, les paramètres de bruit étant déterminés à partir de la distribution de paramètres.

5. Procédé mis en œuvre par ordinateur selon l'une quelconque des revendications 1 à 4, dans lequel un premier paramètre de bruit correspondant à un modèle de bruit spécifique à un premier niveau de lecture a une valeur différente d'un deuxième paramètre de bruit correspondant, qui correspond à un modèle de bruit spécifique à un deuxième niveau de lecture.

6. Procédé mis en œuvre par ordinateur selon l'une quelconque des revendications 1 à 5, dans lequel, pour chaque niveau de lecture, le ou les paramètres de bruit déterminés comprennent une moyenne de la distribution de bruit conditionnée par la profondeur de séquençage stratifiée du niveau de lecture.

7. Procédé mis en œuvre par ordinateur selon l'une quelconque des revendications 1 à 6, dans lequel la sortie générée de chaque modèle de bruit est le ou les paramètres de bruit conditionnés par la profondeur de séquençage stratifiée déterminée pour le niveau de lecture.

8. Procédé mis en œuvre par ordinateur selon l'une quelconque des revendications 1 à 7, dans lequel la sortie générée de chaque modèle de bruit comprend une probabilité qu'un nombre de variants stratifiés pour le niveau de lecture dépasse un seuil.

9. Procédé mis en œuvre par ordinateur selon l'une quelconque des revendications 1 à 8, dans lequel la combinaison des sorties de modèle de bruit générées comprend la combinaison d'un nombre moyen de variants et d'une variance de chaque sortie de modèle de bruit pour produire un nombre moyen de variants global et le paramètre de dispersion globale représentatif d'une distribution de bruit globale pour le résultat combiné.

10. Procédé mis en œuvre par ordinateur selon l'une quelconque des revendications 1 à 9, dans lequel la combinaison des sorties de modèle de bruit générées pour produire le résultat combiné comprend en outre :
la sommation des probabilités des combinaisons identifiées pour déterminer un complément statistique ; et
la détermination d'une valeur de probabilité par soustraction du complément statistique de 1,0.

11. Procédé mis en œuvre par ordinateur selon l'une quelconque des revendications 1 à 10, comprenant en outre :
la réception d'un échantillon de fluide corporel d'un individu ;
la réalisation du séquençage d'ADN sur l'ADNcf de l'échantillon de liquide corporel ;
la génération de lectures de séquence brutes sur la base d'un résultat du séquençage d'ADN ; et
le regroupement et la fusion des lectures de séquences brutes pour générer la pluralité de lectures de séquences traitées.

12. Procédé mis en œuvre par ordinateur selon l'une quelconque des revendications 1 à 11, dans lequel le séquençage d'ADN comprend une opération de séquençage massivement parallèle de l'ADN.

13. Procédé mis en œuvre par ordinateur selon l'une quelconque des revendications 1 à 12, comprenant en outre :
la fourniture, sur la base du résultat combiné, d'un diagnostic d'un sujet présentant un variant.

14. Procédé mis en œuvre par ordinateur selon la revendication 13, dans lequel le variant est choisi dans le groupe constitué par : ACVR1B, AKT3, AMER1, APC, ARID1A, ARID1B, ARID2, ASXL1, ASXL2, ATM, ATR, BAP1 BCL2, BCL6, BCORL1, BCR, BLM, BRAF, BRCA1, BTG1, CASP8, CBL, CCND3, CCNE1, CD74, CDC73, CDK12, CDKN2A, CHD2, CJD2, CREBBP, CSF1R, CTCF, CTNNB1, DICER1, DNAJB1, DNMT1, DNMT3A, DNMT3B, DOT1L, EED, EGFR, EIF1AX, EP300, EPHA3, EPHA5, EPHB1, ERBB2, ERBB4, ERCC2, ERCC3, ERCC4, ESR1, FAM46C, FANCA, FANCC, FANCD2, FANCE, FAT1, FBXW7, FGFR3, FLCN, FLT1, FOXO1, FUBP1, FYN, GATA3, GPR124, GRIN2A, GRM3, H3F3A, HIST1H1C, IDH1, IDH2, IKZF1, IL7R, INPP4B, IRF4, IRS1, IRS2, JAK2, KAT6A, KDM6A, KEAP1, KIF5B, KIT, KLF4, KLH6, KMT2C, KRAS, LMAP1, LRP1B, LZTR1, MAP3K1, MCL1, MGA, MSH2, MSH6, MST1R, MTOR, MYD88, NPM1, NRAS, NTRK1, NTRK2, NUP93, NUTM1, PAX3, PAX8, PBRM1, PGR, PHOX2B, PIK3CA, POLE, PTCH1, PTEN, PTPN11, PTPRT, RAD21, RAF1, RANBP2, RB1, REL, RFWD2, RHOA, RPTOR, RUNX1, RUNX1T1, SDHA, SHQ1, SLIT2, SMAD4, SMARCA4, SMARCD1, SNCAIP, SOCS1, SPEN, SPTA1, SUZ12, TET1, TET2, TGFBR et TNFRSF14.
